# EUROPEAN PATENT SPECIFICATION

(11) **EP 1 711 184 B1**
(45) Date of publication and mention of the grant of the patent: **18.07.2007**
(21) Application number: 04815480.1
(22) Date of filing: 22.12.2004
(51) Int. Cl.: A61K 31/498, A61K 31/4985, C07D 413/12, C07D 403/12, C07D 417/12, C07D 409/12, C07D 407/12, C07D 471/04, C07D 241/44, A61P 3/10

(54) **(3-OXO-3,4-DIHYDRO-QUINOXALIN-2-YL-AMINO)-BENZAMIDE DERIVATIVES AND RELATED COMPOUNDS AS GLYCOGEN PHOSPHORYLASE INHIBITORS FOR THE TREATMENT OF DIABETES AND OBESITY**
(3-OXO-3,4-DIHYDRO-CHINOXALIN-2-YL-AMINO)-BENZAMID-DERIVATE UND VERWANDTE VERBINDUNGEN ALS GLYKOGEN-PHOSPHORYLASE-HEMMER ZUR BEHANDLUNG VON DIABETES UND ADIPOSITAS
DERIVES DE (3-OXO-3, 4-DIHYDRO-QUINOXALIN-2-YL-AMINO)-BENZAMIDE ET COMPOSES AFFERENTS COMME INHIBITEURS DE LA GLYCOGENE PHOSPHORYLASE DANS LE TRAITEMENT DU DIABETE ET DE L'OBESITE

(30) Priority: 06.01.2004 US 534534 P
(43) Date of publication of application: 18.10.2006
(73) Proprietor: JANSSEN PHARMACEUTICA N.V., 2340 Beerse (BE)
(72) Inventor: BEAVERS, Mary, Pat, New Hope, Pennsylvania 18938 (US); DUDASH, Joseph, Hillsborough, New Jersey 08844 (US); ZHANG, Yongzheng, Hillsborough, New Jersey 08844 (US)
(74) Representative: Fisher, Adrian John
(86) International application number: PCT/US2004/043409
(87) International publication number: WO 2005/067932

(56) References cited:
- WO-A-00/42026
- WO-A-01/23389
- WO-A-95/09159
- WO-A-99/46260

## Description

### Background of the Invention

Non-insulin dependent diabetes mellitus (NIDDM) is a polygenic disease characterized by defects in the action of insulin on numerous target tissues including muscle, adipose, and liver. It is well established that normal glucose homeostasis requires precise metabolic control in all of the insulin-responsive tissues; however, the relative importance of each of these tissues to the pathogenesis of NIDDM remains unclear.

More specifically, maintenance of normal glucose homeostasis involves a series of highly orchestrated events mediated by numerous cellular proteins including those which catalyze 1) the transport and phosphorylation of glucose, 2) the assembly of glycogen from the phosphorylated glucose monosaccharide, and 3) glycogenolysis and hepatic glucose production. Each of these processes represents a possible site for therapeutic intervention. There are several pathways that regulate hepatic glucose production (HGP) relating to the regulation of circulating blood glucose levels in NIDDM. Since hepatic glucose production is significantly increased in Type II diabetic patients relative to non-diabetics Consoli, A. (1992) Diabetes Care 15: 430-44, Gerich J.E. (1992) Horm. Metab. Res. 26: 18-21, agents which reduce hepatic glucose output should be beneficial for the treatment of diabetes. Moreover, it has been shown that after a meal, normal suppression of HGP is not observed in Type II diabetics (Gerich, 1992). Therefore, it appears that unregulated hepatic glucose production in diabetic patients contributes to elevated plasma glucose levels.

Glucagon and insulin each play major regulatory roles in glucose metabolism mediated by the liver. In response to a reduction in blood glucose, the alpha cells of the pancreas secrete glucagons, which in turn binds to its cell surface receptor on the liver. Glucagon receptor activation promotes protein kinase A-dependent phosphorylation of phosphorylase kinase. This, in turn, causes the phosphorylation of phosphorylase *b*; thereby converting glycogen phosphorylase to its active a form. Glycogen is converted to Gle-1-P and ultimately glucose to restore the plasma glucose to normal levels. When plasma glucose levels increase, insulin promotes glucose uptake in skeletal muscle and fat, and glycogen synthesis in the liver. In addition, phosphorylase-dependent glycogen breakdown is also blocked by insulin (Bollen, M., and Stalmans,) (1992) Crit. Rev. Biochem. Mol. Biol. 27:227-281). Therefore, the liver serves as an important buffer for the plasma glucose level and glycogen phosphorylase is positioned at a critical juncture for the reciprocal regulation of glucose metabolism.

Glycogen phosphorylase exists in two interconvertible forms: the dephosphorylated inactive form (phosphorylase *b*) and the phosphorylated active form (phosphorylase a) (reviewed by Newgard et al. (1989) Crit. Rev. Biochem. Mol. Biol. 24: 69-99). In resting muscle, glycogen phosphorylase is inactive (a form) and can be activated either by non-covalent cooperative binding of AMP or by covalent phosphorylation. Phosphorylase *a* does not require AMP for activation, althought addition of AMP can produce a 10-20% increase in activity (Oikonomakos et al., 1999) Protein Sci. 8: 1930-1945. Both forms can exist in a less active T-state and a more active R- state (Monod et al., (1965) (J. Mol. Biol. 12: 88-118). The T-state is stabilized by the binding of ATP, Glc-1-P, glucose and caffeine whereas the R-state is induced by AMP substrates and analogues (Johnson et al., (1989), In *Allosteric enzymes.* Boca Raton, FL: CRC Press, 81-127; Oikonomakos et al., 1992, and Johnson, L.N. (1992) FASEB J. 6: 2274-2282).

Caffeine has also been shown to regulate glycogen phosphorylase activity by binding to the purine inhibitory (I) site (Kasvinsky et al., (1978) J. Biol. Chem. 253: 3343-3351). Ercan-Fang et al., (1997) (J. Pharmacol. Exp. Ther. 280: 1312-1318). Although some glucose and purine nucleoside phosphorylase inhibitors reportedly inhibit glycogenolysis in rodent cells and tissues, none of these compounds maintain oral activity in vivo (Kasvinsky et al., (1981) Can. J. Biochem. 59: 387-395; Board, M. et al., (1995a) Eur. J. Biochem. 228: 753-761; Board, et al., (1995b) Biochem. J. 311: 845-852).

Attempts to modulate hepatic glucose production (HGP) with gluconeogenesis inhibitors have yielded limited success. Agents that suppress gluconeogenesis *in vitro* or in diabetic rodents have not been clinically efficacious or safe in humans (Yki-Javinen, (1994) Diabetes Nutr. Metab. 7: 109-119; Bressler and Johnson, (1992) Diabetes Care 15: 792-805). These compounds inhibit gluconeogenesis by reducing gluconeogenic substrates or fatty acid metabolism (Bressler and Johnson, (1992). With the exception of Metformin, an antidiabetic agent with multiple effects including gluconeogenesis inhibition, most inhibitors have failed to reduce HGP and plasma glucose levels in humans caused by hepatic autoregulation, a compensatory increase in hepatic glycogenolysis that maintains a high rate of HGP (Yki-Javinen, 1994). The alternative approach to inhibit glycogenolysis to reduce HGP has not been thoroughly explored. The rate-limiting step of glucose production from the degradative phosphorolysis of glycogen to glucose-l-phosphate (Glc-1-P) is catalysed by glycogen phosphorylase (GP).

In short, glycogen phosphorylase has been implicated in the regulation of excessive hepatic glucose production - a significant contributor to diabetic hyperglycemia. Compounds which specifically inhibit glycogen phosphorylase should reduce blood glucose levels in the post-absorptive state; thereby providing adjunctive therapy for Type II (2) Diabetes. Administration of a selective agent which will specifically inhibit glycogen phosphorylase should result in reduced hepatic glucose production and increased deposition of glycogen in skeletal muscle with a concomitant reduction of blood glucose. The hypoglycemic effect of specific glycogen phosphorylase inhibitors would be advantageous for combination therapy.

### Summary of the Invention

The invention features pharmaceutically active quinoxalinones of formula (I), compositions containing them, and methods of making and using them. 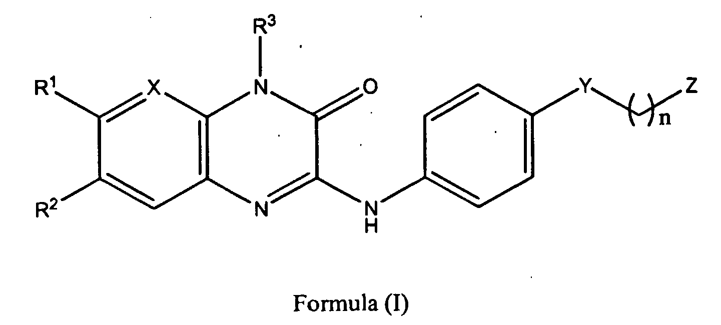 wherein R¹ is H, C₁₋₆ alkyl, or halo;
R² is H or halo;
R³ is H, C ₁₋₆ alkyl,
X is N or CH;
Y is a covalent bond, -NHCO- or -CONH-;
Z is phenyl or a 5 or 6-membered heterocyclyl with between 1 and 2 heteroatoms independently selected from N, O, and S; and
n is 0, 1 or 2;
or a pharmaceutically acceptable salt, ester, amide, hydrate, or solvate thereof.

The invention also features pharmaceutical compositions including one or more compounds of formula (I) and a pharmaceutically acceptable carrier or vehicle.

Another aspect of the invention is the use of a compound of formula (I) for the manufacture of a medicament for treating a disease or condition mediated by glycogen phosphorylase, said method including administering to a patient in need of treatment an effective amount of a pharmaceutical composition which comprises a compound of formula (I). Such diseases or conditions include Type II diabetes and obesity.

Other features and advantages of the invention will become apparent from the following disclosure and the appended claims.

### Detailed Description

### A. Terms

The following terms are defined below and by their usage throughout this disclosure.

"Alkyl" includes optionally substituted straight chain and branched hydrocarbons with at least one hydrogen removed to form a radical group. Alkyl groups include methyl, ethyl, propyl, isopropyl, butyl; isobutyl, t-butyl, 1-methylpropyl, pentyl, isopentyl, sec-pentyl, hexyl, heptyl, octyl, and so on. Alkyl includes cycloalkyl, such as cyclopropyl, cyclobutyl, cyclopentyl, and cyclohexyl.

"Alkoxy" includes an optionally substituted straight chain or branched alkyl group with a terminal oxygen linking the alkyl group to the rest of the molecule. Alkoxy includes methoxy, ethoxy, propoxy, isopropoxy, butoxy, t-butoxy, pentoxy and so on. "Aminoalkyl", "thioalkyl", and "sulfonylalkyl" are analogous to alkoxy, replacing the terminal oxygen atom of alkoxy with, respectively, NH (or NR), S, and SO₂. Heteroalkyl includes alkoxy, aminoalkyl, thioalkyl, and so on.

"Aryl" includes phenyl, naphthyl, biphenylyl, tetrahydronaphthyl, indenyl, and so on, any of which may be optionally substituted. Aryl also includes arylalkyl groups such as benzyl, phenethyl, and phenylpropyl. Aryl includes a ring system containing an optionally substituted 6-membered carbocyclic aromatic ring, said system may be bicyclic, bridge, and/or fused. The system may include rings that are aromatic, or partially or completely saturated. Examples of ring systems include indenyl, pentalenyl, 1-4-dihydronaphthyl, indanyl, benzimidazolyl, benzothiophenyl, indolyl, benzofuranyl, isoquinolinyl, and so on.

"Heterocyclyl" includes optionally substituted aromatic and nonaromatic rings having carbon atoms and at least one heteroatom (O, S, N) or heteroatom moiety (SO₂, CO, CONH, COO) in the ring. Unless otherwise indicated, a heterocyclyl may have a valence connecting it to the rest of the molecule through a carbon atom, such as 3-furyl or 2-imidazolyl, or through a heteroatom, such as N-piperidyl or 1-pyrazolyl. Preferably a monocyclic heterocyclyl has between 5 and 7 ring atoms, or between 5 and 6 ring atoms; there may be between 1 and 5 heteroatoms or heteroatom moieties in the ring, and preferably there are between 1 and 3, or between 1 and 2, or there may be 1 heteroatom or heteroatom moiety. A heterocyclyl may be saturated, unsaturated, aromatic (e.g., heteroaryl), nonaromatic, or fused.

"Heterocyclyl" also includes fused, e.g., bicyclic, rings, such as those optionally condensed with an optionally substituted carbocyclic or heterocyclic five- or six-membered aromatic ring. For example, "heteroaryl" includes an optionally substituted six-membered heteroaromatic ring containing 1, 2 or 3 nitrogen atoms condensed with an optionally substituted five- or six-membered carbocyclic or heterocyclic aromatic ring. Said heterocyclic five- or six-membered aromatic ring condensed with the said five- or six-membered aromatic ring may contain 1, 2 or 3 nitrogen atoms where it is a six-membered ring, or 1, 2 or 3 heteroatoms selected from oxygen, nitrogen and sulfur where it is a five-membered ring.

Examples of heterocyclyls include thiazoylyl, furyl, thienyl, pyranyl, isobenzofuranyl, pyrrolyl, imidazolyl, pyrazolyl, isothiazolyl, isoxazolyl, pyridyl, pyrazinyl, pyrimidinyl, pyridazinyl, indolizinyl, isoindolyl, indolyl, indazolyl, purinyl, quinolyl, furazanyl, pyrrolidinyl, pyrrolinyl, imidazolidinyl, imidazolinyl, pyrazolidinyl, pyrazolinyl, piperidinyl, piperazinyl, indolinyl, and morpholinyl. For example, preferred heterocyclyls or heterocyclic radicals include morpholinyl, piperazinyl, pyrrolidinyl, pyridyl, cyclohexylimino, thienyl,and more preferably, piperidinyl or morpholinyl.

Examples illustrating heteroaryl are thienyl, furanyl, pyrrolyl, imidazolyl, isoxazolyl, oxazolyl, thiazolyl, benzothienyl, benzofuranyl, benzimidazolyl, benzoxazolyl, benzothiazolyl.

"Acyl" refers to a carbonyl moiety attached to either a hydrogen atom (i.e., a formyl group) or to an optionally substituted alkyl or alkenyl chain, or heterocyclyl.

"Halo" or "halogen" includes fluoro, chloro, bromo, and iodo, and preferably fluoro or chloro as a substituent on an alkyl group, with one or more halo atoms, such as trifluoromethyl, trifluoromethoxy, trifluoromethylthio, difluoromethoxy, or fluoromethylthio.

"Pharmaceutically acceptable salts, esters, and amides" or "salts, esters, amides, hydrates, and solvates" include carboxylate salts, amino acid addition salts, esters, and amides which are within a reasonable benefit/risk ratio, pharmacologically effective and suitable for contact with the tissues of patients without undue toxicity, irritation, or allergic response. These salts (or esters, and/or amides) may be, for example, C₁₋₈ alkyl, C₃₋₈ cycloalkyl, aryl, C₂₋₁₀ heteroaryl, or C₂₋₁₀ non-aromatic heterocyclic salts (or esters, and/or amides). Representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, and laurylsulfonate. These may include alkali metal and alkali earth cations such as sodium, potassium, calcium, and magnesium, as well as non-toxic ammonium, quaternary ammonium, and amine cations such as tetramethyl ammonium, methylamine, trimethylamine, and ethylamine. See example, S.M. Berge, et al., "Pharmaceutical Salts," J. Pharm. Sci., 1977, 66:1-19. Representative pharmaceutically acceptable amides of the invention include those derived from ammonia, primary C₁₋₆ alkyl amines and secondary di (C₁₋₆ alkyl) amines. Secondary amines include 5- or 6-membered heterocyclic or heteroaromatic ring moieties containing at least one nitrogen atom and optionally between 1 and 2 additional heteroatoms. Preferred amides are derived from ammonia, C₁₋₃ alkyl primary amines, and di (C₁₋₂ alkyl)amines. Representative pharmaceutically acceptable esters of the invention include C₁₋₇ alkyl, C₅₋₇ cycloalkyl, phenyl, and phenyl(C₁₋₆)alkyl esters. Preferred esters include methyl and ethyl esters.

"Patient" or "subject" includes mammals such as humans and animals (dogs, cats, horses, rats, rabbits, mice, non-human primates) in need of observation, experiment, treatment or prevention in connection with the relevant disease or condition. Preferably, the patient or subject is a human.

"Composition" includes a product comprising the specified ingredients in the specified amounts as well as any product that results from combinations of the specified ingredients in the specified amounts.

"Therapeutically effective amount" or "effective amount" means that amount of active compound or pharmaceutical agent that elicits the biological or medicinal response in a tissue system, animal or human that is being sought by a researcher, veterinarian, medical doctor or other clinician, which includes alleviation of the symptoms of the condition or disorder being treated.

Concerning the various radicals in this disclosure and in the claims, three general remarks are made. The first remark concerns valency. As with all hydrocarbon radicals, whether saturated, unsaturated or aromatic, and whether or not cyclic, straight chain, or branched, and also similarly with all heterocyclic radicals, each radical includes substituted radicals of that type and monovalent, bivalent, and multivalent radicals as indicated by the context of the claims. The context will indicate that the substituent is an alkylene or hydrocarbon radical with at least two hydrogen atoms removed (bivalent) or more hydrogen atoms removed (multivalent). An example of a bivalent radical linking two parts of the molecule is Y in formula (I) which links a phenyl to the -(CH₂)ₙ-Z moiety.

Second, alkyl and heterocyclyl groups as defined herein are understood to include substituted alkyl and heterocyclyl groups. Hydrocarbyls include monovalent radicals containing carbon and hydrogen such as alkyl, alkenyl, alkynyl, cycloalkyl, and cycloalkenyl (whether aromatic or unsaturated), as well as corresponding divalent (or multi-valent) radicals such as alkylene, alkenylene, phenylene, and so on. Heterocarbyls include monovalent and divalent (or multi-valent) radicals containing carbon, optionally hydrogen, and at least one heteroatom. Examples of monovalent heterocarbyls include acyl, acyloxy, alkoxyacyl, heterocyclyl, heteroaryl, aroyl, benzoyl, dialkylamino, hydroxyalkyl, and so on. Using "alkyl" as an example, "alkyl" should be understood to include substituted alkyl having one or more substitutions, such as between 1 and 5, 1 and 3, or 2 and 4 substituents. Preferably, an alkyl group has between 1 and 3 substituents. The substituents may be the same (dihydroxy- or dimethyl-substituted), similar (chlorofluoro-substituted), or different (chloro- or aminomethyl-substituted). Examples of substituted alkyl include haloalkyl (such as fluoromethyl, chloromethyl, difluoromethyl, perchloromethyl, 2-bromoethyl, trifluoromethyl, and 3-iodocyclopentyl), hydroxyalkyl (such as hydroxymethyl, hydroxyethyl, 2-hydroxypropyl), aminoalkyl (such as aminomethyl, 2-aminoethyl, 3-aminopropyl, and 2-aminopropyl), nitroalkyl, alkylalkyl, and so on. A di(C ₁₋₆ alkyl)amino group includes independently selected alkyl groups, to form, for example, methylpropylamino and isopropylmethylamino, in addition dialkylamino groups having two of the same alkyl group such as dimethyl amino or diethylamino. Similarly, "heterocyclyl" is understood to be substituted

Third, only stable compounds are intended.

Compounds of the invention are further described in the next section.

### B. Compounds

The invention features compounds of formula (I). Examples of such compounds include those compounds of formula (I) wherein: (a) R¹ is H; (b) R² is H; (c) R³ is H or C₁₋₃ alkyl; (d) n is 0 or 1; (e) Y is -CONH-; (f) R³ is an alkyl substituted with hydroxy, carboxy, dimethylamino, piperidinyl, pyridyl, or morpholinyl; (g) Z is selected from phenyl, furanyl, thiophenyl, isoxazolyl, pyridazyl, pyrazolyl, N-methyl-pyrazolyl, pyrimidyl, pyrrolyl, N-methyl-pyrrole, imidazolyl, pyridyl, oxazolyl, and thiazolyl; (h) Z is selected from furanyl, thiophenyl, isoxazolyl, and pyrazolyl; (i) R¹ is H; R² is H; and R³ is H or C₁₋₂ alkyl; (j) n is 0 or 1; and Y is -CONH-; (k) Z is selected from furanyl, thiophenyl, isoxazolyl, pyridazyl, pyrazolyl, pyrimidyl, pyrrolyl, imidazolyl, pyridyl, oxazolyl, and thiazolyl; (1) limitations of (k) and n is 0 or 1; (m) limitations of (i) and (j); (n) limitations of (g), (i), and (j); (o) limitations of (e) and (i); and combinations of the above.

Examples of preferred compounds where Y=CONH include:
N-Isoxazol-3-yl-4-(4-Methyl-3-oxo-3,4-dihydro-quinoxalin-2ylamino)-benzamide;
4-(4-Methyl-3-oxo-3,4-dihydro-quinoxalin-2ylamino)-N-thiophen-2-ylmethyl-benzamide;
N-Isoxazol-5-yl-4-(4-Methyl-3-oxo-3,4-dihydro-quinoxalin-2ylamino)-benzamide;
4-(4-Methyl-3-oxo-3,4-dihydro-quinoxalin-2ylamino)-N-furan-2-ylmethyl-benzamide;
N-Isoxazol-3-yl-4-(4-Ethyl-3-oxo-3,4-dihydro-quinoxalin-2ylamino)-benzamide;
4-(7-Fluoro-4-methyl-3-oxo-3,4-dihydro-quinoxalin-2ylamino)-N-thiophen-2-ylmethyl-benzamide; and
4-(4-Methyl-3-oxo-3,4-dihydro-pyrido[2,3-b]pyrazin-2-ylamino)-N-thiophen-2-ylmethyl-benzamide.
4-(4-Ethyl-3-oxo-3,4-dihydro-quinoxalin-2ylamino)-N-thiophen-2-ylmethyl-benzamide;
4-(4-Methyl-3-oxo-3,4-dihydro-quinoxalin-2ylamino)-N-(1*H*-pyrazol-3-yl)-benzamide;
N-Isoxazol-3-yl-4-[4-(2-Morpholin-4-yl-ethyl)-3-oxo-3,4-dihydro-quinoxalin-2ylamino]-benzamide;
4-(4,6-Dimethyl-3-oxo-3,4-dihydro-quinoxalin-2ylamino)-N-thiophen-2-ylmethyl-benzamide;
4-[4-(2-hydroxy-ethyl)-3-oxo-3,4-dihydro-quinoxalin-2ylamino)]-N-thiophen-2-ylmethyl-benzamide;
N-Isoxazol-3-yl-4-(4-Methyl-3-oxo-3,4-dihydro-pyrido[2,3-b]pyrazin-2-ylamino)-benzamide;
4-[4-(2-morpholin-4-yl-ethyl)-3-oxo-3,4-dihydro-quinoxalin-2ylamino)]-N-thiophen-2-ylmethyl-benzamide; and
4-[4-(2-piperidin-1-yl-ethyl)-3-oxo-3,4-dihydro-quinoxalin-2ylamino)]-N-thiophen-2-ylmethyl-benzamide.

Examples of preferred compounds where Y=NHCO include:
Isoxazole-5-carboxylic acid [4-(4-Methyl-3-oxo-3,4-dihydro-quinoxalin-2ylamino)-phenyl]-amide;
Isoxazole-3-carboxylic acid [4-(4-Methyl-3-oxo-3,4-dihydro-quinoxalin-2ylamino)-phenyl]-amide; and
*N*-[4-(4-Methyl-3-oxo-3,4-dihydro-quinoxalin-2ylamino)-phenyl]-nicotinamide.

More preferred compounds where Y is a covalent bond include:
1-Methyl-3-[4-(1H-pyrrol-2-yl)-phenylamino]-1*H*-quinoxalin-2-one; and
3-(4-imidazol-1-yl-phenylamino)-1-methyl-1*H*-quinoxalin-2-one.

Additional preferred compounds include:
N-Isoxazol-3-yl-4-(7-Fluoro-4-methyl-3-oxo-3,4-dihydro-quinoxalin-2ylamino)-benzamide;
4-(6-Fluoro-4-methyl-3-oxo-3,4-dihydro-quinoxalin-2ylamino)-N-thiophen-2-ylmethyl-benzamide;
N-Isoxazol-3-yl-4-(6-Fluoro-4-methyl-3-oxo-3,4-dihydro-quinoxalin-2ylamino)-benzamide;
N-Isoxazol-3-yl-4-(6-Chloro-4-methyl-3-oxo-3,4-dihydro-quinoxalin-2ylamino)-benzamide;
N-Isoxazol-3-yl-4-(7-Chloro-4-methyl-3-oxo-3,4-dihydro-quinoxalin-2ylamino)-benzamide;
4-(6-Chloro-4-methyl-3-oxo-3,4-dihydro-quinoxalin-2ylamino)-N-thiophen-2- ylmethyl-benzamide;
4-(7-Chloro-4-methyl-3-oxo-3,4-dihydro-quinoxalin-2ylamino)-N-thiophen-2- ylmethyl-benzamide;
N-Isoxazol-3-yl-4-[4-(2-piperidin-1-yl-ethyl)-3-oxo-3,4-dihydro-quinoxalin-2ylamino]-benzamide;
4-(4-Ethyl-3-oxo-3,4-dihydro-pyrido[2,3-b]pyrazin-2-ylamino)-N-thiophen-2-ylmethyl-benzamide;
N-Isoxazol-3-yl-4-(4-ethyl-3-oxo-3,4-dihydro-pyrido[2,3-b]pyrazin-2-ylamino)-benzamide;
N-Isoxazol-3-yl-4-[7-fluoro-4-(2-Morpholin-4-yl-ethyl)-3-oxo-3,4-dihydro-quinoxalin-2ylamino]-benzamide;
4-[7-Fluoro-4-(2-piperidin-1-yl-ethyl)-3-oxo-3,4-dihydro-quinoxalin-2ylamino)]-N-thiophen-2-ylmethyl-benzamide;
4-(4-Methyl-3-oxo-3,4-dihydro-quinoxalin-2ylamino)-N-oxazol-2-yl-benzamide;
N-Furan-2-yl-4-(4-Methyl-3-oxo-3,4-dihydro-quinoxalin-2ylamino)-benzamide;
4-(4-Methyl-3-oxo-3,4-dihydro-quinoxalin-2ylamino)-N-thiophen-2-yl-benzamide;
4-(4-Methyl-3-oxo-3,4-dihydro-quinoxalin-2ylamino)-N-(1*H*-pyrrol-3-yl)-benzamide;
4-(4-Methyl-3-oxo-3,4-dihydro-quinoxalin-2ylamino)-N-(1-methyl-1*H-*pyrrol-3-yl)-benzamide;
4-(4-Methyl-3-oxo-3,4-dihydro-quinoxalin-2ylamino)-N-(pyrimidin-2-yl)-benzamide;
N-(1*H*-Imidazol-2-yl)-4-(4-methyl-3-oxo-3,4-dihydro-quinoxalin-2ylamino)-benzamide;
4-[4-(2-pyrrolidin-1-yl-ethyl)-3-oxo-3,4-dihydro-quinoxalin-2ylamino)]-N-thiophen-2-ylmethyl-benzamide; and
N-Isoxazol-3-ylmethyl-4-(4-methyl-3-oxo-3,4-dihydro-quinoxalin-2ylamino)-benzamide.

### C. Synthesis

The compounds of the invention can be made according to both conventional synthetic organic methods, as well as combinatorial or matrix synthetic methods. The following three schemes and remarks are exemplified in Examples 1-78.

### General Synthetic Scheme for Quinoxalinones where Y=CONH

Synthesis of analogues where Y=CONH begins with either alkylation of the appropriately substituted 2-nitro-aniline or addition of a substituted amine to the appropriately substituted 2-fluoro-nitrobenzene. The nitro group is reduced to the amine, and the diamine is treated with diethyl oxalate to give the quinoxalindione. Reaction of the dione with phosphorous oxychloride gives the chloroimidate. Addition of methyl 4-amino-benzoate to the chloroimidate followed by amide formation with the appropriate amine gives the desired compounds.

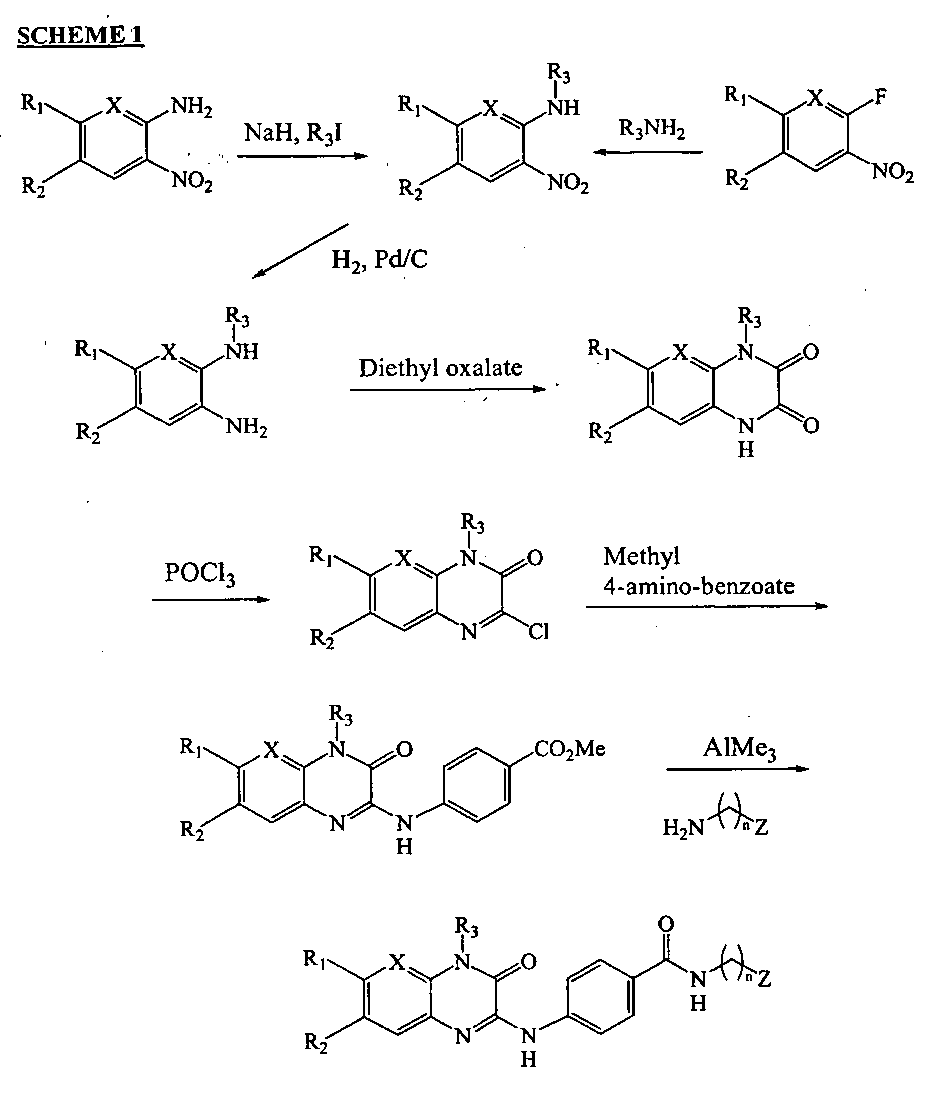

### General Synthetic Scheme for Quinoxalinones where Y= NHCO

The chloroimidate for analogues where Y=NHCO is synthesized in a manner similar to that which was described earlier. Addition of 1,4-diamino-benzene to the chloroimidate followed by reaction with the appropriate acid chloride gives the desired compounds, as shown in Scheme 2.

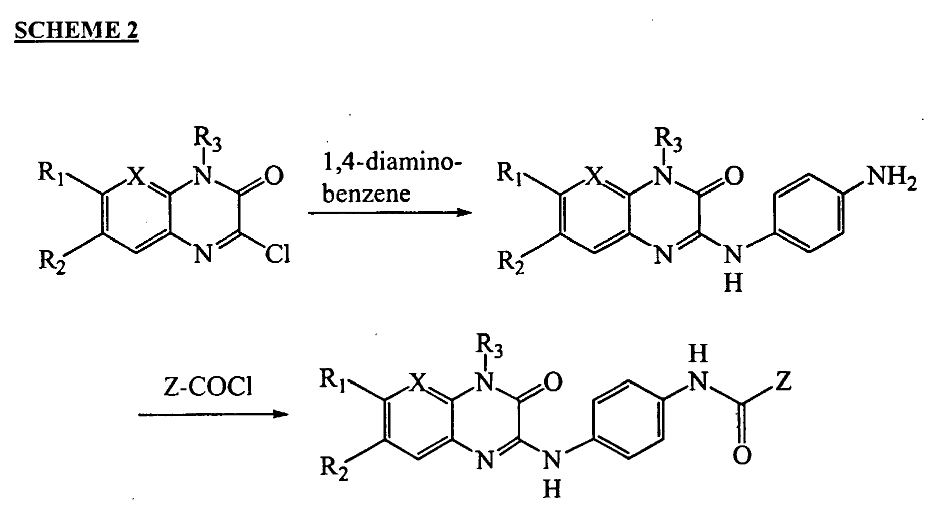

### General Synthetic Scheme for Quinoxalinones where Y= Covalent Bond

The chloroimidate for analogues where Y=NHCO is synthesized in a manner similar to that which was described earlier. Addition of 4-iodo-aniline followed by palladium catalyzed coupling with the appropriate boronic acid or stannane gives the desired compound. Alternatively, the appropriately 4-substituted aniline can be added to the chloroimidate to give the desired compound in a single operation. (Scheme 3)

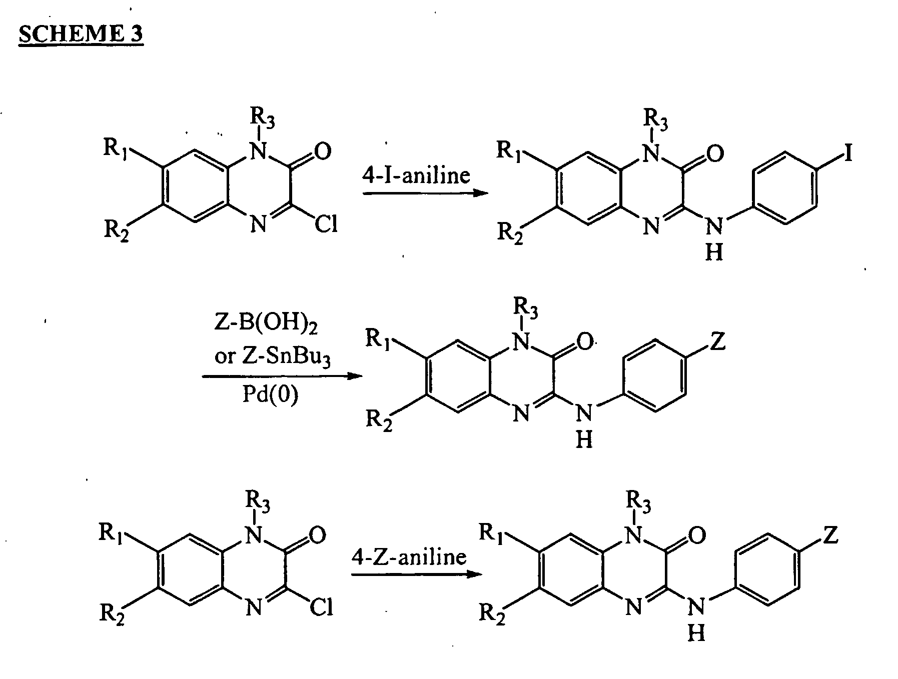

### D. Formulation and Administration

The compounds of the present invention may be formulated into various pharmaceutical forms for administration purposes. To prepare these pharmaceutical compositions, an effective amount of a particular compound, for example, in base or acid addition salt form, as the active ingredient is intimately mixed with a pharmaceutically acceptable carrier.

A carrier may take a wide variety of forms depending on the form of preparation desired for administration. These pharmaceutical compositions are desirably in unitary dosage form suitable, preferably, for oral administration or parenteral injection. For example, in preparing the compositions in oral dosage form, any of the usual pharmaceutical media may be employed. These include water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. In view of their ease in administration, tablets and capsules represent the most advantageous oral dosage unit form, in which case solid pharmaceutical carriers are generally employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Such additives may facilitate the administration to the skin and/or may be helpful for preparing the desired compositions. These compositions may be administered in various ways, e.g., as a transdermal patch, as a spot-on, as an ointment. Acid addition salts of the compounds of formula I, due to their increased water solubility over the corresponding base form, are more suitable in the preparation of aqueous compositions.

It is especially advantageous to formulate the aforementioned pharmaceutical compositions in dosage unit form for ease of administration and uniformity of dosage. Dosage unit form as used in the specification herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Pharmaceutically acceptable acid addition salts include the therapeutically active non-toxic acid addition salt forms, which the disclosed compounds are able to form. The latter can conveniently be obtained by treating the base form with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic, maleic, fumaric, malic, tartaric, citric, methanesulfonic, ethanesulfonic, benzenesulfonic, p-toluenesulfonic, cyclamic, salicylic, p-aminosalicylic, palmoic and the like acids. The term addition salt also comprises the solvates which the disclosed componds, as well as the salts thereof, are able to form. Such solvates are for example hydrates, alcoholates and the like. Conversely the salt form can be converted by treatment with alkali into the free base form.

Stereoisomeric forms define all the possible isomeric forms that the compounds of formula (I) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the (R)- or (S)-configuration; substituents on bivalent cyclic saturated radicals may have either the cis- or trans-configuration. The invention encompasses stereochemically isomeric forms including diastereoisomers, as well as mixtures thereof in any proportion of the disclosed compounds. The disclosed compounds may also exist in their tautomeric forms. Such forms although not explicitly indicated in the above and following formulae are intended to be included within the scope of the present invention.

Those of skill in the treatment of disorders or conditions mediated by glycogen phosphorylase could easily determine the effective daily amount from the test results presented hereinafter and other information. In general it is contemplated that a therapeutically effective dose would be from 0.001 mg/kg to 5 mg/kg body weight, more preferably from 0.01 mg/kg to 0.5 mg/kg body weight. It may be appropriate to administer the therapeutically effective dose as two, three, four or more sub-doses at appropriate intervals throughout the day. Said sub-doses may be formulated as unit dosage forms, for example, containing 0.05 mg to 250 mg or 750 mg, and in particular 0.5 to 50 mg of active ingredient per unit dosage form. Examples include 2 mg, 4 mg, 7 mg, 10 mg, 15 mg, 25 mg, and 35 mg dosage forms. Compounds of the invention may also be prepared in time-release or subcutaneous or transdermal patch formulations. Disclosed compound may also be formulated as a spray or other topical or inhalable formulations.

The exact dosage and frequency of administration depends on the particular compound of formula (I) used, the particular condition being treated, the severity of the condition being treated, the age, weight and general physical condition of the particular patient as well as other medication the patient may be taking, as is well known to those skilled in the art. Furthermore, it is evident that said effective daily amount may be lowered or increased depending on the response of the treated patient and/or depending on the evaluation of the physician prescribing the compounds of the instant invention. The effective daily amount ranges mentioned herein are therefore only guidelines.
The next section includes detailed information relating to the use of the disclosed compounds and compositions.

### E. Uses

The compounds and compositions of the invention are pharmaceutically active. For example, they may be used to treat glycogen phosphorylase-mediated conditions and diseases. These disease include NIDDM or Type II (2) Diabetes, hyperglycemia (particularly in a post-absorptive state).

### F. Examples

### Compound 1:

### 1-Methyl-1,4-dihydro-quinoxaline-2,3-dione

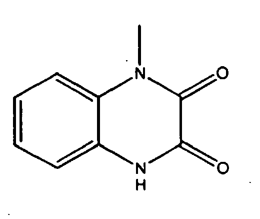

### Method A:

To a solution of N-methyl-1,2-phenylenediamine (5g, 41 mmol) in dichloromethane (80 mL) at 0° C was added triethylamine (11.4 mL, 82 mmol) followed by dropwise addition ethyl chlorooxoacetate (4.6 mL, 41 mmol). The mixture was stirred at room temperature for 2h, then at 60°C for 2h. The mixture was cooled and the precipitate that formed was collected by vacuum filtration, washed with hexanes and dried to give a light pink solid.

### Compound 2:

### 3-Chloro-1-methyl-1H-quinoxalin-2-one

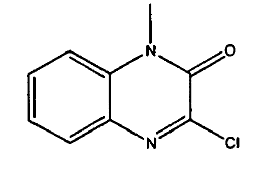

### Method B:

To a suspension of compound 1 (3g, 17 mmol) in toluene (30 mL) was added diisopropylethyl amine (2.4 mL, 13.6 mmol) followed by phosphorous oxychloride (2.4 mL, 25.6 mmol). The mixture was stirred at 110°C for 3h. The solvent was removed *in vacuo,* and the resulting dark residue was dissolved in dichloromethane, washed with cold saturated sodium bicarbonate solution, dried (magnesium sulfate), and passed thru a short column of silica gel. The solution was concentrated *in vacuo* to give a white solid.

### Compound 3:

### 4-(4-Methyl-3-oxo-3,4-dihydro-quinoxalin-2ylamino)-benzoic acid methyl ester

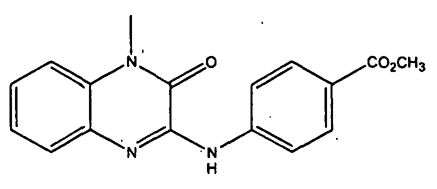

### Method C:

To a solution of compound 2 (1.4 g, 7.3 mmol) in acetonitrile (20 mL) was added methyl 4-aminobenzoate (1 g, 6.6 mmol). The reaction was stirred at 80°C overnight. The precipitated solid was collected by vacuum filtration, washed with saturated sodium bicarbonate solution and water, and dried *in vacuo* to give a white solid.

### Compound 4:

### N-Isoxazol-3-yl-4-(4-Methyl-3-oxo-3,4-dihydro-quinoxalin-2ylamino)-benzamide

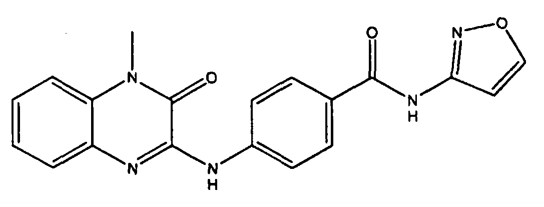

### Method D:

To a solution of trimethylaluminum (2M in hexanes, 0.62 mmol, 0.31 mL) in toluene (1.5 mL) at room temperature was added 3-aminoisoxazole (0.31 mmol, 26 mg) in toluene (0.5 mL) dropwise via syringe. After 30 min, compound 3 was added and the reaction was heated to reflux for 5h. The mixture was cooled, quenched with 1N hydrochloric acid solution, and the mixture is extracted with ethyl acetate. The combined organic layers are washed with brine and dried (magnesium sulfate). After removal of solvent *in vacuo* purification of the resulting solid on silica gel (15% ethyl acetate/ dichloromethane) gave a white solid. ¹H NMR (DMSO) δ 11.29 (s, 1H), 9.81 (s, 1H), 8.85 (d, J=1.3 Hz, 1H), 8.34 (d, J=8.7 Hz, 2H), 8.06 (d, J=8.9 Hz, 2H), 7.64 (d, J=7.6 Hz, 1H), 7.50 (d, J=8.3 Hz, 1H), 7.43-7.31 (m, 2H), 7.07 (d, J=1.3 Hz, 1H), 3.72 (s, 3H).

### Compound 5:

### 4-(4-Methyl-3-oxo-3,4-dihydro-quinoxalin-2ylamino)-N-(1H-pyrazol-3-yl)-benzamide

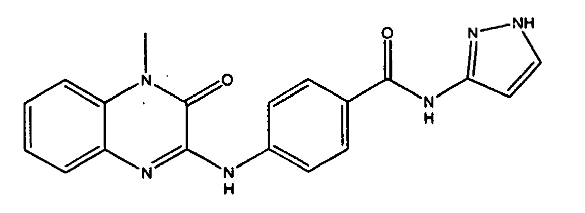

Compound 5 was obtained as a yellow solid from 3-aminopyrazole and compound 3 by method D. ¹H NMR (DMSO) δ 12.41 (s, 1H), 10.65 (s, 1H), 9.74 (s, 1H), 8.30 (d, J=8.6 Hz, 2H), 8.03 (d, J=8.6 Hz, 2H), 7.63 (m, 1H), 7.50 (m, 1H), 7.43-7.30 (m, 2H), 6.65 (bs, 1H), 3.72, (s, 3H).

### Compound 6:

### 4-(4-Methyl-3-oxo-3,4-dihydro-quinoxalin-2ylamino)-N-thiazol-2-yl-benzamide

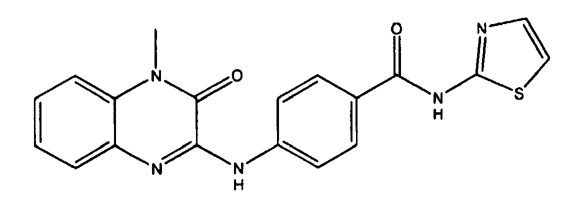

Compound 6 was obtained as a yellow solid from 3-aminothiazole and compound 3 by method D. ¹H NMR (DMSO) δ 12.48 (s, 1H), 9.84 (s, 1H), 8.35 (d, J=8.8 Hz, 2H), 8.13 (d, J=8.8 Hz, 2H), 7.65 (dd, J=7.7, 1.6 Hz, 1H), 7.56 (d, J=3.6 Hz, 1H), 7.51 (m, 1H), 7.44-7.31 (m, 2H), 7.27 (d, J=3.4 Hz, 1H), 3.72 (s, 3H).

### Compound 7:

### N-Isoxazol-5-yl-4-(4-Methyl-3-oxo-3,4-dihydro-quinoxalin-2ylamino)-benzamide

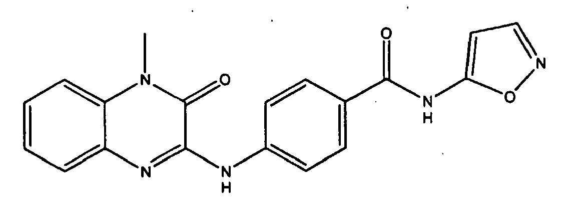

Compound 7 was obtained as a yellow solid from 5-aminoisoxazole and compound 3 by method D. ¹H NMR (DMSO) δ 11.89 (s, 1H), 9.84 (s, 1H), 8.51 (d, J=1.8 Hz, 1H), 8.36 (d, J=8.8 Hz, 2H), 8.06 (d, J=8.9 Hz, 2H), 7.64 (dd, J=7.6, 1.2 Hz, 1H), 7.51 (d, J=7.9 Hz, 1H), 7.43-7.31 (m, 2H), 6.43 (d, J=1.9 Hz, 1H), 3.72 (s, 3H).

### Compound 8:

### 4-(4-Methyl-3-oxo-3,4-dihydro-quinoxalin-2ylamino)-N-(1H-pyrazol-3-yl)-benzamide

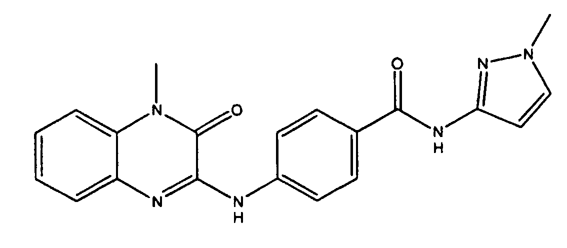

Compound 8 was obtained as a white solid from 3-amino-1-methylpyrazole and compound 3 by method D. ¹H NMR (DMSO) δ 10.66 (s, 1H), 9.74 (s, 1H), 8.28 (d, J=8.7 Hz, 2H), 8.02 (d, J=8.8 Hz, 2H), 7.62 (m, 2H), 7.50 (m, 1H), 7.42-7.31 (m, 2H), 6.60 (d, J=2.3 Hz, 1H), 3.79 (s, 3H), 3.72 s, 3H)

### Compound 9:

### 4-(4-Methyl-3-oxo-3,4-dihydro-quinoxalin-2ylamino)-N-thiophen-3-yl-benzamide

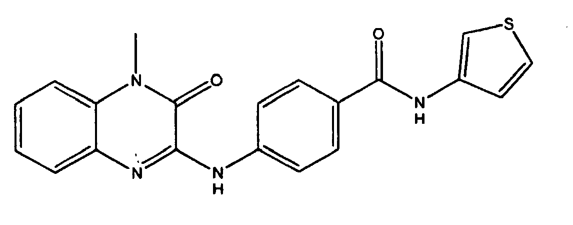

Compound 9 was obtained from 3-aminothiophene and compound 3 by method D as a yellow solid (37 mg). ¹H NMR (DMSO) δ 10.53 (s, 1H), 9.74 (s, 1H), 8.30 (d, J=9.0 Hz, 2H), 7.97 (d, J=9.0 Hz, 2H), 7.71 (dd, J=3.2, 1,2 Hz, 1H), 7.60 (dd, J= 7.7, 1.6 Hz, 1H), 7.49-7.30 (m, 5H), 3.7 (s, 3H).

### Compound 10:

### 4-(4-Methyl-3-oxo-3,4-dihydro-quinoxalin-2ylamino)-N-thiophen-2-ylmethyl-benzamide

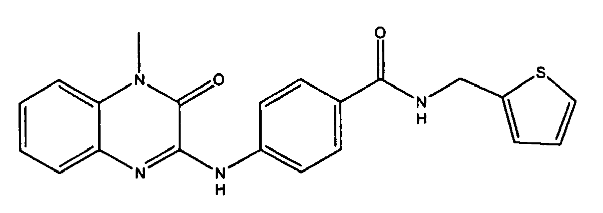

Compound 10 was obtained as a white solid from 2-aminomethyl-thiophene and compound 3 by method D. ¹H NMR (DMSO) δ 9.70 (s, 1H), 9.01 (t, J=5.7 Hz, 1H), 8.26 (d, J=8.7 Hz, 2H), 7.89 (d, J=8.77 Hz, 2H), 7.61 (dd, J= 7.7, 1.5 Hz, 1H), 7.49 (dd, J=8.2, 1.3 Hz, 1H), 7.41-7.29 (m, 3H), 7.03, (m, 1H), 6.97 (dd, J=5.0, 3.4 Hz, 1H), 4.64 (d, J=5.8 Hz, 2H), 3.71 (s, 3H).

### Compound 11:

### 4-(4-Methyl-3-oxo-3,4-dihydro-quinoxalin-2ylamino)-N-furan-2-ylmethyl-benzamide

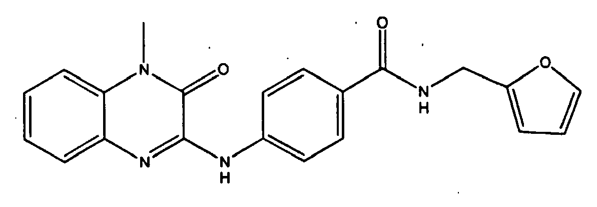

Compound 11 was obtained as a white solid from 2-aminomethyl-furan and compound 3 by method D. ¹H NMR (DMSO) δ 9.69 (s, 1H), 8.84 (t, J=6.2 Hz, 1H), 8.26 (d, J=8.7 Hz, 2H), 7.89 (d, J=8.7 Hz, 2H), 7.60 (m, 1H), 7.49 (dd, J=8.2, 1.2 Hz, 1H), 7.41-7.29 (m, 2H), 6.40, (dd, J=3.2, 1.9 Hz, 1H), 6.28 (dd, J=3.2, 0.7 Hz, 1H), 4.47 (d, J=5.6 Hz, 2H), 3.71 (s, 3H).

### Compound 12:

### 4-(4-Methyl-3-oxo-3,4-dihydro-quinoxalin-2ylamino)-N-pyrazin-2-yl-benzamide

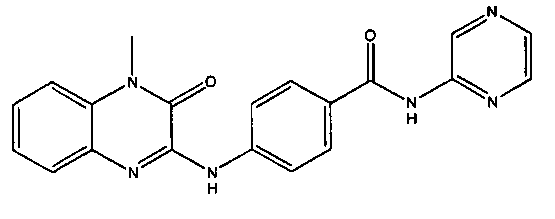

Compound 12 was obtained as a white solid from aminopyrazine and compound 3 by method D. ¹H NMR (DMSO) δ 11.69 (s, 1H), 10.97 (s, 1H), 9.82 (s, 1H), 9.44 (d, J=1.6 Hz, 1H), 8.48 (m, 1H), 8.41 (d, J=2.5 Hz, 1H), 8.34 (d, J=8.8 Hz, 2H), 8.09 (d, J=8.8 Hz, 2H), 7.64 (dd, J= 7.7, 1.7 Hz, 1H), 7.51 (dd, J=8.2, 1.2 Hz, 1H), 7.43-7.31 (m, 2H), 3.72 (s, 3H).

### Compound 13:

### 3-(4-amino-phenylamino)-1-methyl-1H-quinoxalin-2-one

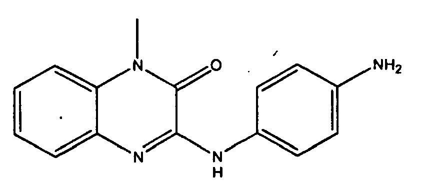

Compound 13 was obtained as a white solid from 1,4-diaminobenzene and compound 2 by method C.

### Compound 14:

### Isoxazole-5-carboxylic acid [4-(4-Methyl-3-oxo-3,4-dihydro-quinoxalin-2ylamino)-phenyl]-amide

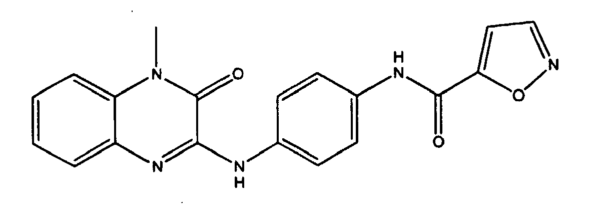

### Method E:

To a suspension of compound 13 (50 mg, 0.19 mmol) in dichloromethane (2 mL) at room temperature was added pyridine (23 µL, 0.28 mmol) followed by isoxazole-5-carbonyl chloride (25 mg, 0.19 mmol). After 30 min, the precipitated solid was collected by vacuum filtration, washed with water and dichloromethane, and dried *in vacuo* to give compound 14 as a white solid. ¹H NMR (DMSO) δ 10.73 (s, 1H), 9.52 (s, 1H) 8.82 (d, J=1.9 Hz, 1H), 8.17 (d, J= 9 Hz, 2H), 7.75 (d, J=9 Hz, 2H), 7.59 (dd, J=7.6, 1.5 Hz; 1H), 7.47 (m, 1H), 7.38-7.27 (m, 2H), 7.25 (d, J=1.7 Hz, 1H), 3.71 (s, 3H).

### Compound 15:

### Isoxazole-3-carboxylic acid [4-(4-Methyl-3-oxo-3,4-dihydro-quinoxalin-2ylamino)-phenyl]-amide

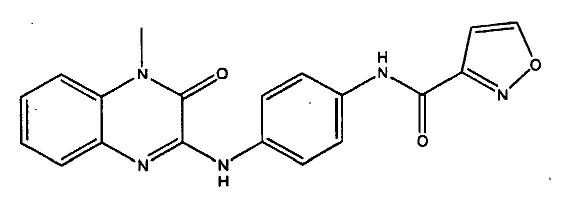

Compound 15 was obtained as a white solid from compound 13 and isoxazole-3-carbonyl chloride by method E. ¹H NMR (DMSO) δ 10.69 (s, 1H), 9.50 (s, 1H) 9.16 (d, J=1.7 Hz, 1H), 8.16 (d, J= 9.1 Hz, 2H), 7.77 (d, J=9.1 Hz, 2H), 7.59 (dd, J=8.7, 1.7 Hz, 1H), 7.46 (dd, J= 8.2, 1.4 Hz, 1H), 7.38-7.27 (m, 2H), 7.03 (d, J=1.7 Hz, 1H), 3.71 (s, 3H).

### Compound 16:

### N-[4-(4-Methyl-3-oxo-3,4-dihydro-quinoxalin-2ylamino)-phenyl]-nicotinamide

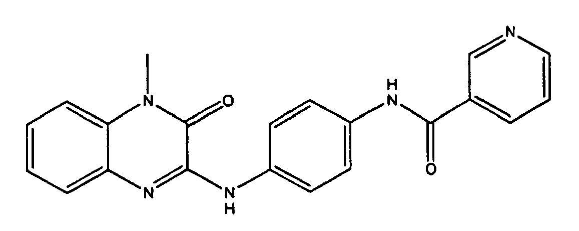

Compound 16 was obtained as a white solid from compound 13 and nicotinoyl chloride by method E. ¹H NMR (DMSO) δ 10.66 (s, 1H), 9.54 (s, 1H), 9.27 (d, J=1.3 Hz, 1H), 8.89 (dd, J=5.1, 1.5 Hz, 1H), 8.61 (m, 1H), 8.17 (d, J=8.9 Hz, 2H), 7.83 (m, 1H), 7.79 (d, J=8.9 Hz, 2H), 7.59 (dd, J=7.3, 1.9 Hz, 1H), 7.47 (m, 1H), 7.38-7.27 (m, 2H), 3.71 (s, 3H)

### Compound 17:

### Thiophene-2-carboxylic acid [4-(4-Methyl-3-oxo-3,4-dihydro-quinoxalin-2ylamino)-phenyl]-amide

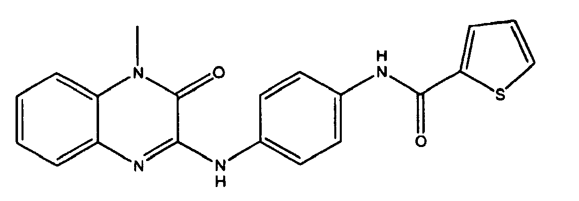

Compound 17 was obtained as a white solid from compound 13 and thiophene-2-carbonyl chloride by method E. ¹H NMR (DMSO) δ 10.21 (s, 1H), 9.47 (s, 1H), 8.14 (d, J=9 Hz, 2H), 8.02 (dd, J=3.8, 1.1 Hz, 1H), 7.85 (dd, J=5.1, 1.0 Hz, 1H), 7.70 (d, J= 9 Hz, 2H), 7.58 (dd, J=7.4, 1.8 Hz, 1H), 7.46 (dd, J=8.2, 1.5 Hz, 1H), 7.37-7.26 (m, 2H), 7.23 (dd, J=4.9, 3.8 Hz, 1H), 3.71 (s, 3H).

### Compound 18:

### Furan-2-carboxylic acid [4-(4-Methyl-3-oxo-3,4-dihydro-quinoxalin-2ylamino)-phenyl]-amide

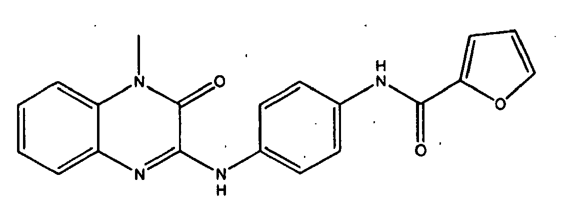

Compound 18 was obtained as a white solid from compound 13 and furan-2-carbonyl chloride by method E. ¹H NMR (DMSO) δ 10.16 (s, 1H), 9.46 (s, 1H), 8.12 (d, J=9.1 Hz, 1H), 7.94 (dd, J=1.7, 0.8 Hz, 1H), 7.73 (d, J= 9 Hz, 2H), 7.58 (dd, J=7.5, 1.6 Hz, 1H), 7.46 (dd, J=8.0, 1.5 Hz, 1H), 7.37-7.26 (m, 3H), 6.71 (dd, J=3.4, 1.7Hz, 1H), 3.71 (s, 3H).

### Compound 19:

### 1-Methyl-1H-pyrrole-2-carboxylic acid [4-(4-Methyl-3-oxo-3,4-dihydro-quinoxalin-2ylamino)-phenyl]-amide

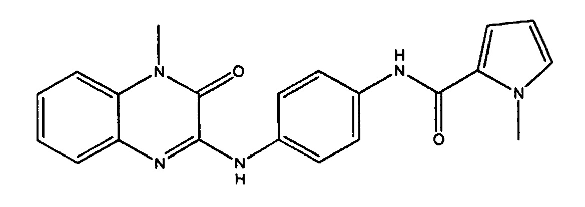

Compound 19 was obtained as a white solid from compound 13 and 1-methylpyrrole-2-carbonyl chloride by method E. ¹H NMR (CDCl₃) δ 8.43 (s, 1H), 7.96 (d, J=9.1 Hz, 2H), 7.68 (m, 1H), 7.59 (d, J=9.1 Hz, 2H0, 7.33-7.24 (m, 3H), 6.79 (m, 1H), 6.71 (m, 1H), 6.16 (m, 1H), 4.00 (s, 3H), 3.78 (s, 3H).

### Compound 20:

### 3-(4-iodo-phenylamino)-1-methyl-1H-quinoxalin-2-one

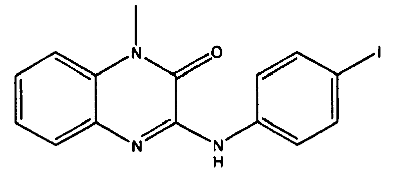

Compound 20 was obtained as a yellow solid from compound 2 and 4-iodo-aniline by method C.

### Compound 21:

### 3-(4-furan-3-yl-phenylamino)-1-methyl-1H-quinoxalin-2-one

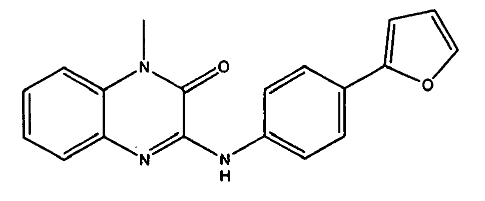

To a degassed solution of compound 20 (100 mg, 0.27 mmol), furan-3-boronic acid (45 mg, 0.4 mmol), and tetrakis(triphenylphosphine) palladium (31 mg, 0.027 mmol) in dimethoxyethane (1.5 mL) at room temperature was added aqueous 2N sodium carbonate solution (1.5 mL). The reaction was stirred under an atmosphere of nitrogen overnight at 95°C. The mixture was cooled and diluted with ethyl acetate. The mixture was washed with saturated sodium bicarbonate solution, water, and brine, dried over sodium sulfate, and concentrated *in vacuo.* After purification on silica gel (50% hexane/dichloromethane), compound 21 was obtained as a yellow solid. ¹H NMR (CDCl₃) δ 8.49 (s, 1H), 7.99 (d, J=8.7 Hz, 2H), 7.73 (m, 2H), 7.52 (d, J=8.7 Hz, 2H), 7.49 (m, 1H), 7.36-7.24 (m, 3H), 6.72 (s, 1H), 3.76 (s, 3H).

### Compound 22:

### 1-Methyl-3-[4-(1H-pyrrol-2-yl)-phenylamino]-1H-quinoxalin-2-one

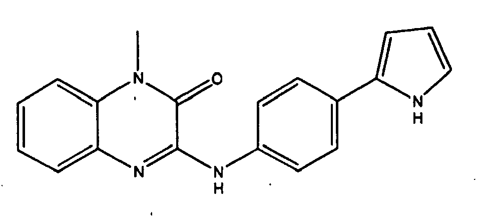

To a degassed solution of compound 20 (50 mg, 0.13 mmol), 1-(t-butoxycarbonyl)pyrrole-2-boronic acid (42 mg, 0.2 mmol), and tetrakis(triphenylphosphine) palladium (16 mg, 0.013 mmol) in dimethoxyethane (1 mL) at room temperature was added aqueous 2N sodium carbonate solution (1 mL). The reaction was stirred under an atmosphere of nitrogen overnight at 95°C. The mixture was cooled and diluted with ethyl acetate. The mixture was washed with saturated sodium bicarbonate solution, water, and brine, dried over sodium sulfate, and concentrated *in vacuo.* After purification on silica gel (50% hexane/ dichloromethane), the resulting yellow solid is heated at 185°C for 20 min. The residue was purified on silica gel (10% ethyl acetate/ dichloromethane) to give compound 22 as a yellow solid. ¹H NMR (CDCl₃) δ 8.48 (s, 1H), 8.43 (bs, 1H), 7.99 (d, J=8.8 Hz, 2H), 7.70 (m, 1H), 7.51 (d, J=8.8 Hz, 2H), 7.51 (d, J=8.6 Hz, 1H), 7.36-7.24 (m, 3H), 6.87 (m, 1H), 6.65 (m, 1H), 6.31 (m, 1H), 3.78 (s, 3H).

### Compound 23:

### 3-(4-imidazol-1-yl-phenylamino)-1-methyl-1H-quinoxalin-2-one

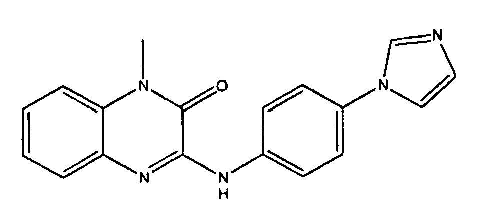

Compound 23 was obtained as a yellow solid from compound 2 and 4-(1*H*-imidazol-1-yl) aniline by method C. ¹H NMR (CDCl₃) δ 8.55 (s, 1H), 8.10 (d, J=8.9 Hz, 2H), 7.85 (bs (1H), 7.73-7.70 (m, 1H), 7.42 (d, J=8.9 Hz, 2H), 7.38-7.26 (m, 4H), 7.22 (bs, 1H), 3.80 (s, 3H).

### Compound 24:

### N-ethyl-1,2-phenylenediamine

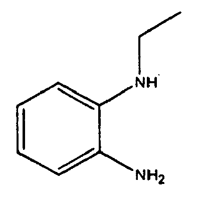

### Method F:

To a solution of 1-ethyl-2-nitro-aniline (2g, 12.1 mmol) in ethanol (40 mL) was added 10% palladium on carbon (500 mg). The mixture was reacted in a Paar apparatus under 50 psi of hydrogen gas for 2h. The mixture was filtered thru celite, the celite was washed with ethyl acetate, and the combined organic solvents were concentrated *in vacuo.* Compound 24 was obtained as a yellow oil.

### Compound 25:

### 1-Ethyl-1,4-dihydro-quinoxaline-2,3-dione

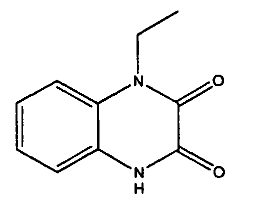

Compound 25 was obtained as a white solid from compound 24 by method A.

### Compound 26:

### 3-Chloro-1-ethyl-1H-quinoxalin-2-one

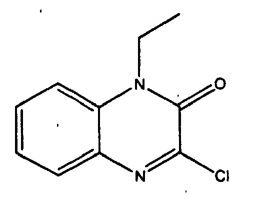

Compound 26 was obtained as a gray solid from compound 25 by method B.

### Compound 27:

### 4-(4-Ethyl-3-oxo-3,4-dihydro-quinoxalin-2ylamino)-benzoic acid methyl ester

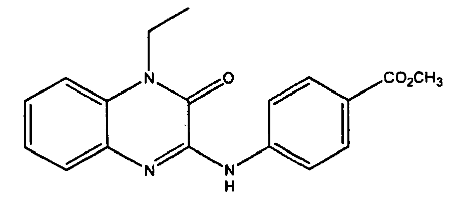

Compound 27 was obtained as a white solid from compound 26 and methyl 4-aminobenzoate by method C.

### Compound 28:

### N-Isoxazol-3-yl-4-(4-Ethyl-3-oxo-3,4-dihydro-quinoxalin-2ylamino)-benzamide

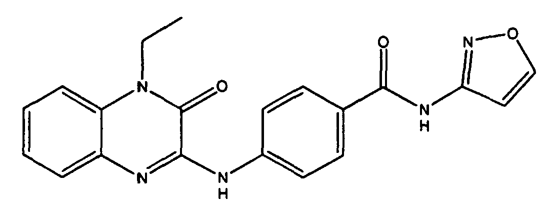

Compound 28 was obtained as a yellow solid from compound 27 by method D. ¹H NMR (DMSO) δ 11.29 (s, 1H), 9.80 (s, 1H), 8.85 (d, J=1.7 Hz, 1H), 8.33 (d, J=9.0 Hz, 2H), 8.06 (d, J=8.9 Hz, 2H), 7.66-7.30 (m, 4H), 7.07 (d, J=1.7 Hz, 1H), 4.36 (q, J=7.1 Hz, 2H), 1.30 (t, J=7.1 Hz, 3H).

### Compound 29:

### 4-(4-Ethyl-3-oxo-3,4-dihydro-quinoxalin-2ylamino)-N-(1H-pyrazol-3-yl)-benzamide

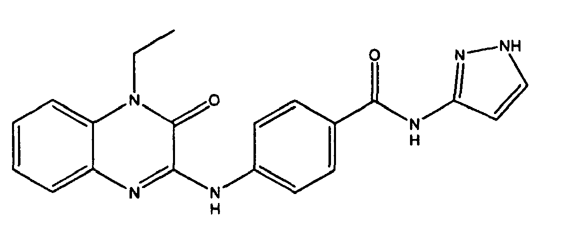

Compound 29 was obtained as a yellow solid from compound 27 by method D. ¹H NMR (DMSO) δ 12.4 (s, 1H), 10.64 (s, 1H), 9.73 (s, 1H), 8.28 (d, J=8.9 Hz, 2H), 8.03 (d, J=8.9 Hz, 2H), 7.60 (m, 2H), 7.56 (m, 1H), 7.42-7.29 (m, 2H), 6.65 (s, 1H), 4.36 (q, J=7.1 Hz, 2H), 1.30 (t, J=7.1 Hz, 3H).

### Compound 30:

### 4-(4-Eihyl-3-oxo-3,4-dihydro-quinoxalin-2ylamino)-N-thiazol-2-yl-benzamide

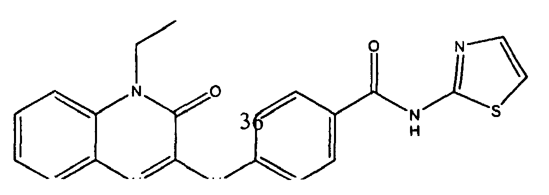

Compound 30 was obtained as a white solid from compound 27 by method D. ¹H NMR (DMSO) δ 12.47 (s, 1H), 9.82 (s, 1H), 8.34 (d, J=8.8 Hz, 2H), 8.12 (d, J=8.8 Hz, 2H), 7.66 (dd, J= 7.7, 1.5 Hz, 1H), 7.56 (m, 2H), 7.44-7.30 (m, 2H), 7.27 (d, J=3.4 Hz, 1H), 4.36 (q, J=6.9 Hz, 2H), 1.31 (t, J=6.9 Hz, 3H).

### Compound 31:

### 4-(4-Ethyl-3-oxo-3,4-dihydro-quinoxalin-2ylamino)-N-thiophen-2-ylmethyl-benzamide

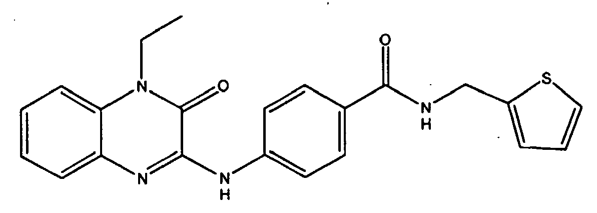

Compound 31 was obtained as a white solid from compound 27 by method D. ¹H NMR (DMSO) δ 9.69 (s, 1H), 9.02 (t, J=6.0 Hz, 1H), 8.25 (d, J=8.9 Hz, 2H), 7.89 (d, J=8.9 Hz, 2H), 7.62 (dd, J= 7.7, 1.5 Hz, 1H), 7.55 (m, 1H), 7.41-7.28 (m, 3H), 7.03, (dd, J=3.4, 1.0 Hz, 1H), 6.96 (dd, J=5.1, 3.4 Hz, 1H), 4.64 (d, J=5.8 Hz, 2H), 4.35 (q, J=6.9 Hz, 2H), 1,29 (t, J=6.9 Hz, 3H).

### Compound 32:

### N²-Methyl-pyridine-2,3-diamine

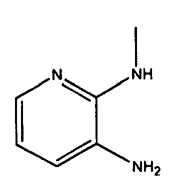

Compound 32 was obtained as an oil from 2-methylamino-3-nitro-pyridine by method F.

### Compound 33:

### 4-Methyl-1,4-dihydro-pyrido[2,3-b]pyrazine-2,3-dione

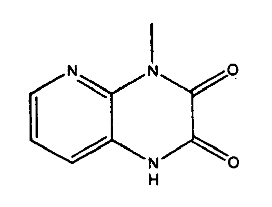

Compound 33 was obtained as a white solid from compound 32 by method A.

### Compound 34:

### 2-Chloro-4-methyl-4H-pyrido[2,3-b]pyrazine-3-one

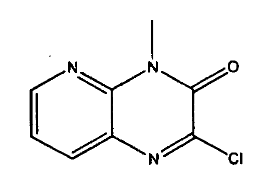

Compound 34 was obtained as a white solid from compound 33 by method B.

### Compound 35:

### 4-(4-Methyl-3-oxo-3,4-dihydro-pyrido[2,3-b]pyrazin-2-ylamino)-N-thiophen-2-ylmethyl-benzamide

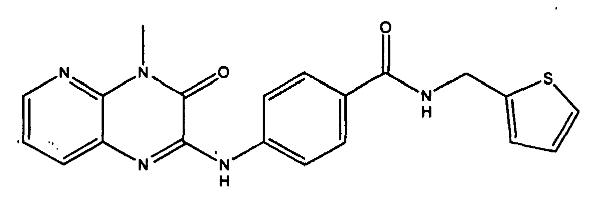

Compound 35 was obtained as a yellow solid from compound 34 and 4-amino-N-thiophen-2ylmethyl-benzamide by method C. ¹H NMR (DMSO) δ 9.86 (s, 1H), 9.04 (t, J=5.6 Hz, 1H), 8.38 (m, 1H), 8.27 (d, J=8.8 Hz, 2H), 7.99 (m, 1H), 7.90 (d, J=8.8 Hz, 2H), 7.39-7.34 (m, 1H), 7.02 (m, 1H), 6.96 (m, 1H), 4.64 (d, J= 5.7 Hz, 2H), 3.75 (s, 3H).

### Compound 36:

### N-Isoxazol-3-yl-4-(4-Methyl-3-oxo-3,4-dihydro-pyrido[2,3-b]pyrazin-2-ylamino)-benzamide

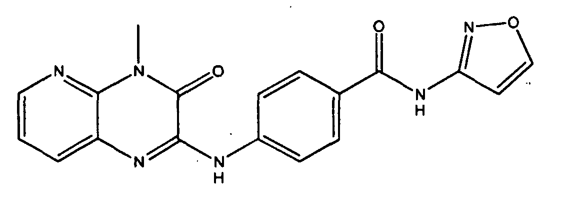

Compound 36 was obtained as a yellow solid from compound 34 and 4-amino-N-isoxazol-3yl-benzamide by method C. ¹H NMR (DMSO) δ 11.31 (s, 1H), 9.96 (s, 1H), 8.85 (d, J=1.7 Hz, 1H), 8.40 (dd, J=4.7, 1.7 Hz, 1H), 8.34 (d, J=8.9 Hz, 2H), 8.06 (d, J=8.9 Hz, 2H), 8.02 (dd, J= 7.8 ,1.6 Hz, 1H), 7.38 (dd, J=7.8, 4.8 Hz, 1H), 7.07 (d, J=1.7 Hz, 1H), 3.76 (s, 3H).

### Compound 37:

### Methyl-(5-methyl-2-nitro-phenyl)-amine

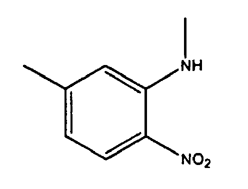

### Method G:

To a suspension of sodium hydride (95%, 684 mg, 27.1 mmol) in DMF (50 mL) at 0°C was added a solution of 5-methyl-2-nitro-aniline (4g, 26.3 mmol) in DMF (40mL). After stirring for 10 minutes, methyl iodide (7.2 mL, 114.8 mmol) was added dropwise over 20 min. The reaction was stirred for 1h at 0°C and then at room temperature for 1h. The reaction was diluted with water, stirred for 10 min, and compound 37 was obtained as a red solid and collected by vacuum filtration.

### Compound 38:

### 4, N²-Dimethyl-benzene-1,2-diamine

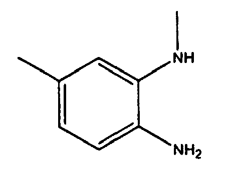

Compound 38 was obtained as an oil from compound 37 by method F.

### Compound 39:

### 1,7-Dimethyl-1,4-dihydro-quinoxaline-2,3-dione

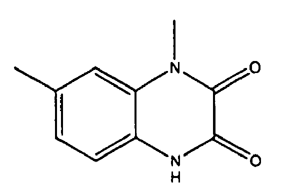

Compound 39 was obtained as a gray solid from compound 38 by method A.

### Compound 40:

### 3-Chloro-1,7-dimethyl-1H-quinoxalin-2-one

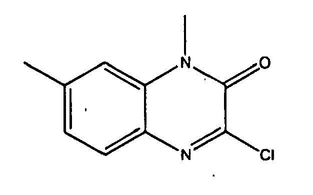

Compound 40 was obtained as a brown solid from compound 39 by method B.

### Compound 41:

### 4-(4,6-Dimethyl-3-oxo-3,4-dihydro-quinoxalin-2ylamino)-N-thiophen-2-ylmethyl-benzamide

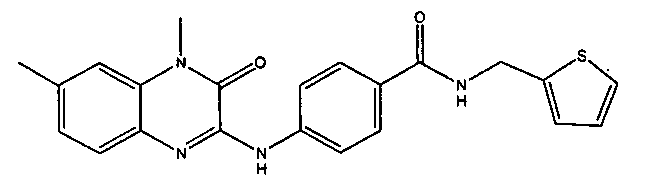

Compound 41 was obtained as a white solid from compound 40 and 4-amino-N-thiophen-2ylmethyl-benzamide by method C. ¹H NMR (DMSO) δ 9.63 (s, 1H), 9.03 (t, J=5.8 Hz, 1H), 8.25 (d, J=8.9 Hz, 2H), 7.89 (d, J=8.9 Hz, 2H), 7.52 (d, J=8.1 Hz, 1H), 7.39 (dd, J=5.21, 1.4 Hz, 1H), 7.33 (s, 1H), 7.16 (dd, J= 8, 1 Hz, 1H), 7.03 (d, J=3.5 Hz, 1H), 6.98 (dd, J= 5.2, 3.5 Hz, 1H), 4.64 (d, J=5.8 Hz, 2H), 3.71 (s, 3H), 2.46 (s, 3H).

### Compound 42:

### (4-Fluoro-2-nitro-phenyl)-methyl-amine

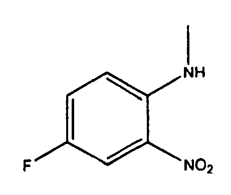

Compound 42 was obtained as a red solid from 4-fluoro-2-nitro-aniline by method G.

### Compound 43:

### 4-Fluoro-N¹-methyl-benzene-1,2-diamine

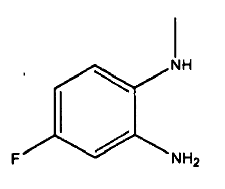

Compound 43 was obtained as an oil from compound 42 by method F.

### Compound 44:

### 6-Fluoro-1-methyl-1,4-dihydro-quinoxaline-2,3-dione

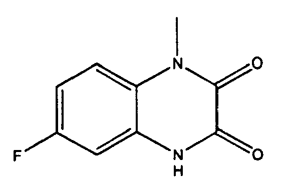

Compound 44 was obtained as a gray solid from compound 43 by method A.

### Compound 45:

### 3-Chloro-6-fluoro-1-methyl-1H-quinoxalin-2-one

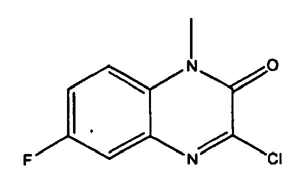

Compound 45 was obtained as a brown solid from compound 44 by method B.

### Compound 46:

### 4-(7-Fluoro-4-methyl-3-oxo-3,4-dihydro-quinoxalin-2ylamino)-N-thiophen-2-ylmethyl-benzamide

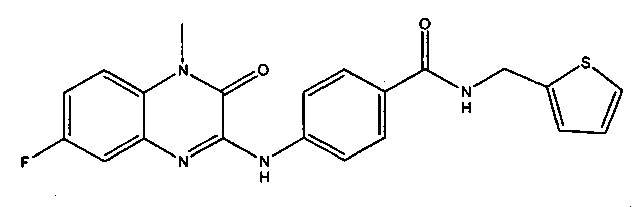

Compound 46 was obtained as a white solid from compound 45 and 4-amino-N-thiophen-2ylmethyl-benzamide by method C. ¹H NMR (DMSO) δ 9.82 (s, 1H), 9.05 (t, J=5.7 Hz, 1H), 8.25 (d, J=8.8 Hz, 2H), 7.89 (d, J=8.8 Hz, 2H), 7.51 (m, 1H), 7.43-7.38 (m, 2H), 7.25 (m, 1H), 7.02 (m, 1H), 7.67 (m, 1H), 4.64 (d, J=5.9 Hz, 2H), 3.70 (s, 3H).

### Compound 47:

### [2-(tert-Butyl-dimethyl-silanoxy)-ethyl]-(2-nitro-phenyl)-amine

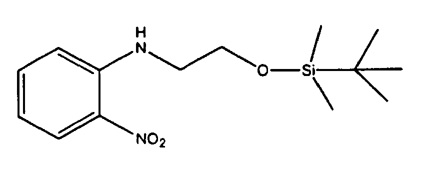

Compound 47 was obtained from 2-nitro-aniline and 2-bromoethoxy *tert*-butyl-dimethylsilane by method G.

### Compound 48:

### N-[(2-tert-butyl-dimethyl-silanyloxy)-ethyl]-benzene-1,2-diamine

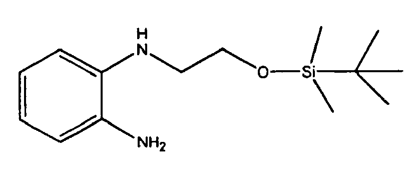

Compound 48 was obtained as a dark solid from compound 47 by method F.

### Compound 49:

### 1-[(2-tert-butyl-dimethyl-silanyloxy)-ethyl]-1,4-dihydro-quinoxaline-2,3-dione

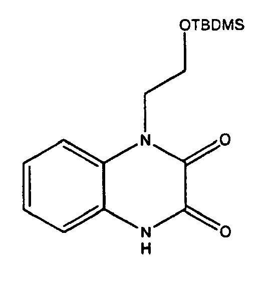

Compound 49 was obtained as a gray solid from compound 48 by method A.

### Compound 50:

### 3-Chloro-1-(2-hydroxy-ethyl)-1H-quinoxalin-2-one

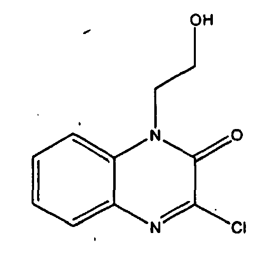

Compound 50 was obtained as a dark solid from compound 49 by method B.

### Compound 51:

### 4-[4-(2-hydroxy-ethyl)-3-oxo-3,4-dihydro-quinoxalin-2ylamino)]-N-thiophen-2-ylmethyl-benzamide

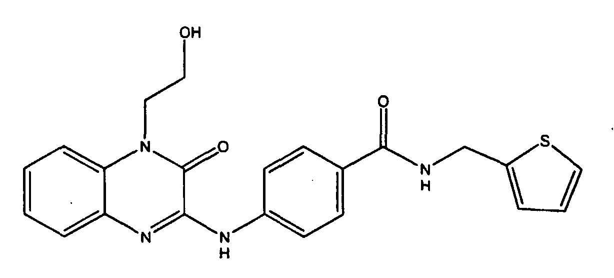

Compound 51 was obtained as a white solid from compound 50 and 4-amino-N-thiophen-2ylmethyl-benzamide by method C. ¹H NMR (DMSO) δ 9.69 (s, 1H), 9.02 (t, J=5.7 Hz, 1H), 8.26 (d, J=8.9 Hz, 2H), 7.88 (d, J=8.9 Hz, 2H), 7.62-7.57 (m, 2H), 7.40-7.27 (m, 3H), 7.03 (dd, J=3.5, 1 Hz, 1H), 6.97 (dd, J= 5.1, 3.5 Hz, 1H), 4.92 (t, J=5.9 Hz, 1H), 4.64 (d, J= 5.7 Hz, 2H), 4.40 (t, J=6.1 Hz, 2H), 3.75 (appq, J=6.1 Hz, 2H)

### Compound 52:

### (2-Morpholin-4-yl-ethyl)-(2-nitro-phenyl)-amine

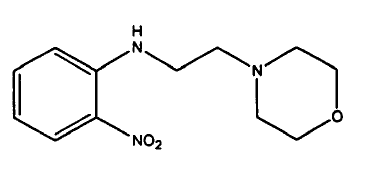

### Method H:

To a mixture of 2-fluoronitrobenzene (6.3 mL, 59.3 mmol) and sodium acetate (138 mg, 1.68 mmol) at room temperature was added 4-(aminoethyl)-morpholine (7.4 mL, 56.1 mmol) dropwise. The reaction was stirred at 80°C for 1h. The reaction was cooled to room temperature, added to water, and extracted with ethyl acetate (2x). The combined organic layers were washed with brine, dried over sodium sulfate, and concentrated *in vacuo.* After column chromatography on silica (5%-15% methanol in dichloromethane), compound 52 was obtained as an orange oil.

### Compound 53:

### 1-(2-Morpholin-4-yl-ethyl)-1,4-dihydro-quinoxaline-2,3-dione

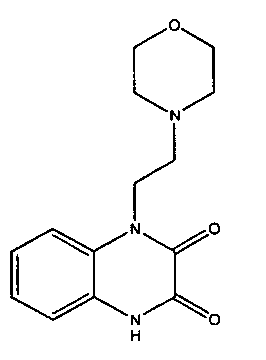

### Method I:

To a solution of compound 52 (8.1 g, 32.3 mmol) in ethanol (55 mL) was added palladium on carbon (10%, 750 mg). The mixture was reacted in a Paar apparatus under 50psi of hydrogen for 2h. The mixture was filtered through Celite, the Celite pad was washed with ethanol, and the solution was concentrated *in vacuo* until the total volume was approximately 50 mL. To this solution was added diethyl oxalate (20 mL) and the mixture was heated to reflux with removal of ethanol by Dean-Stark trap. After 3h, the mixture was cooled to room temperature and the precipitate is collected by vacuum filtration. Compound 53 was obtained as a brown solid.

### Compound 54:

### 3-Chloro-1-(2-Morpholin-4-yl-ethyl)-1H-quinoxalin-2-one

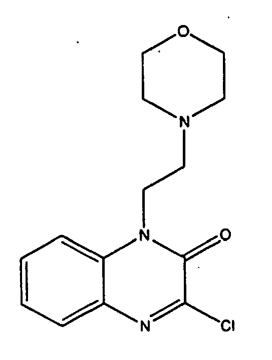

Compound 54 was obtained as the hydrochloride salt from compound 53 by method B.

### Compound 55:

### N-Isoxazol-3-yl-4-[4-(2-Morpholin-4-yl-ethyl)-3-oxo-3,4-dihydro-quinoxalin-2ylamino]-benzamide

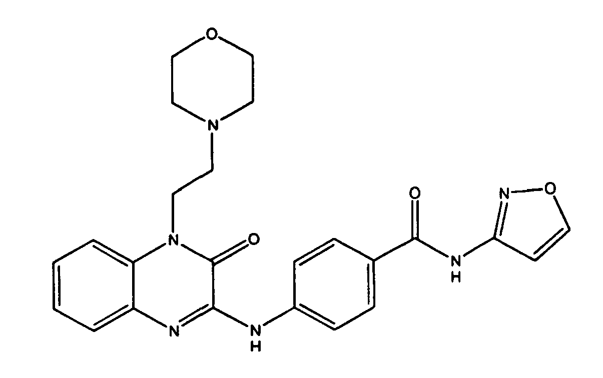

Compound 55 was obtained from compound 54 and 4-amino-N-isoxazol-3yl-benzamide by method C as a trifluoroacetic acid salt after purification by reverse phase HPLC. ¹H NMR (DMSO) δ 11.30 (s, 1H), 9.87 (s, 1H), 8.86 (d, J=1.7 Hz, 1H), 8.36 (d, J=8.9 Hz, 2H), 8.07 (d, J=8.9 Hz, 2H), 7.71 (dd, J= 7.5, 1.8 Hz, 1H), 7.50 (d, J= 8.3 Hz, 1H), 7.48-7.37 (m, 2H), 7.08 (d, J= 1.7 Hz, 1H), 4.73 (m, 2H), 4.15-3.92 (m, 4H), 3.89-3.57 (m, 4H), 3.51-3.21 (m, 2H).

### Compound 56:

### 4-[4-(2-morpholin-4-yl-ethyl)-3-oxo-3,4-dihydro-quinoxalin-2ylamino)]-N-thiophen-2-ylmethyl-benzamide

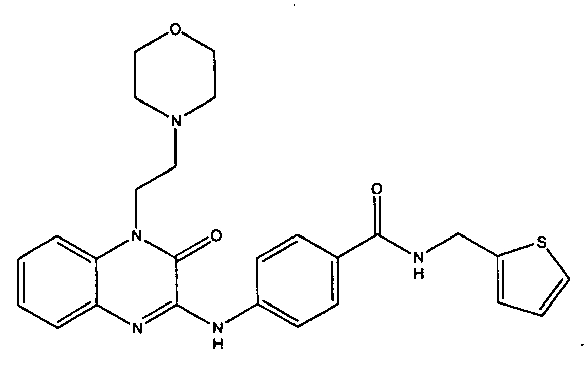

Compound 56 was obtained as a hydrochloride salt from compound 54 and 4-amino-N-thiophen-2ylmethyl-benzamide by method C. ¹H NMR (DMSO) δ 10.68 (bs, 1H), 9.76 (s, 1H), 9.07 (t, J= 5.6 Hz, 1H), 8.29 (d, J= 8.58 Hz, 2H), 7.91 (d, J= 8.58 Hz, 2H), 7.67 (appt, J= 7.5 Hz, 2H), 7.43-7.35 (m, 3H), 7.04 (s, 1H), 6.99-6.95 (m, 1H), 4.77-4.72 (m, 2H), 4.65 (d, J=5.7 Hz, 2H), 4.05-3.95 (m, 2H), 3.78-3.64 (m, 4H), 3.61-3.53 (m, 2H), 3.31-3.24 (m, 2H).

### Compound 57:

### 4-[4-(2-morpholin-4-yl-ethyl)-3-oxo-3,4-dihydro-quinoxalin-2ylamino)]-benzoic acid methyl ester

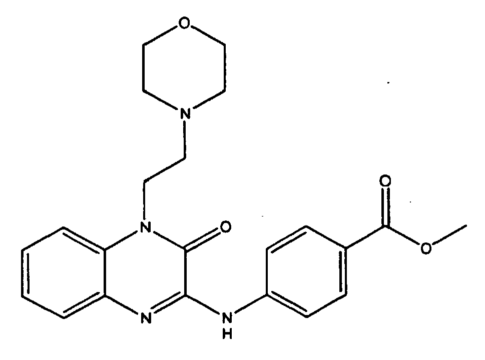

Compound 57 was obtained as a hydrochloride salt from compound 54 and methyl 4-aminobenzoate by method C.

### Compound 58:

### 4-[4-(2-morpholin-4-yl-ethyl)-3-oxo-3,4-dihydro-quinoxalin-2ylamino)]-N-pyrazol-3-yl-benzamide

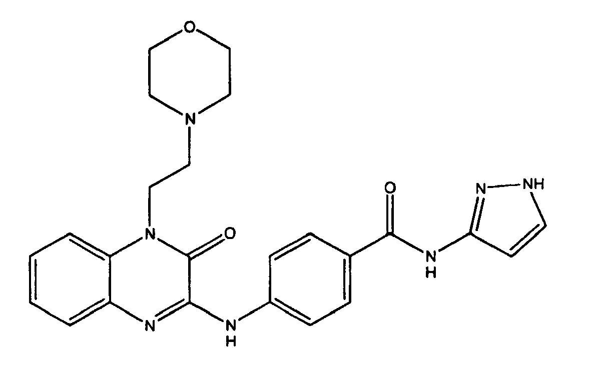

Compound 58 is obtained as a hydrochloride salt from compound 57 and 3-aminopyrazole by method D. ¹H NMR (DMSO) δ 10.7 (s, 1H), 10.34 (bs, 1H), 9.8 (s, 1H), 8.31 (d, J=8.9 Hz, 2H), 8.05 (d, J=8.9 Hz, 2H), 7.70-7.66 (m, 3H), 7.45-7.35 (m, 2H), 6.62 (d, J= 2.3 Hz, 1H), 4.76 (appt, J=5.8 Hz, 2H), 4.10-4.03 (m, 4H), 3.76-3.54 (m, 4H), 3.51-3.32 (m, 2H)

### Compound 59:

### (2-Piperidin-1-yl-ethyl)-(2-nitro-phenyl)-amine

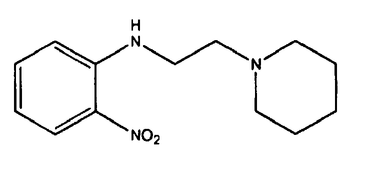

Compound 59 was obtained as an orange oil from 2-fluoronitrobenzene and 1-(aminoethyl)-piperidine by method H.

### Compound 60:

### 1-(2-Piperidin-1-yl-ethyl)-1,4-dihydro-quinoxaline-2,3-dione

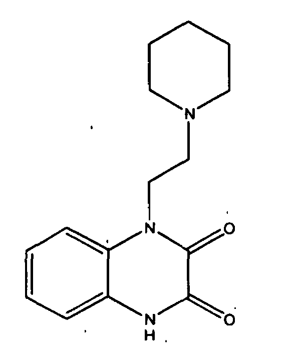

Compound 60 was obtained as a gray solid from compound 59 by method I.

### Compound 61:

### 3-Chloro-1-(2-piperidin-1-yl-ethyl)-1H-quinoxalin-2-one

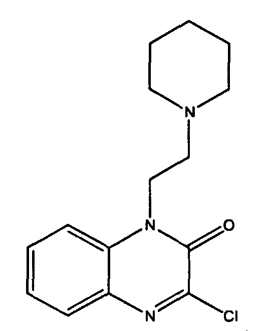

Compound 61 was obtained as a hydrochloride salt from compound 60 by method B.

### Compound 62:

### 4-[4-(2-piperidin-1-yl-ethyl)-3-oxo-3,4-dihydro-quinoxalin-2ylamino)]-N-thiophen-2-ylmethyl-benzamide

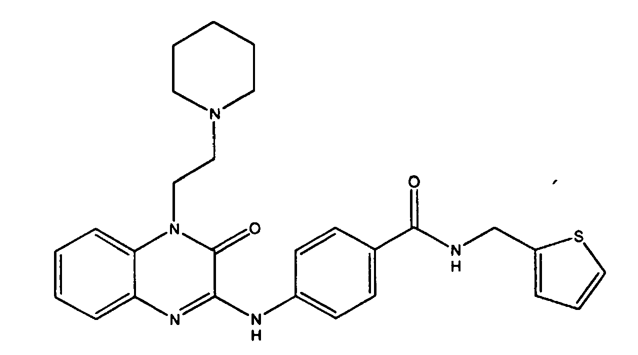

Compound 62 was obtained as a hydrochloride salt from compound 61 and 4-amino-N-thiophen-2ylmethyl-benzamide by method C. ¹H NMR (DMSO) δ 10.38 (bs, 1H), 9.74 (s, 1H), 9.08 (t, J=5.9 Hz, 1H), 8.27 (d, J=8.7 Hz, 2H), 7.90 (d, J=8.7 Hz, 2H), 7.72 (d, J=7.8 Hz, 1H), 7.65 (dd, J=7.6,1.8 Hz, 1H), 7.42-7.33 (m, 3H), 7.03 (d, J=2.6 Hz, 1H), 6.97 (dd, J=4.9, 3.5 Hz, 1H), 4.76 (appt, J=6.5 Hz, 2H), 4.64 (d, J=5.9 Hz, 2H), 3.67 (d, J=11.5 Hz, 2H), 3.39 (d, J=5.8 Hz, 2H), 3.02 (d, J=10.6, 2H), 1.85-1.7 (m, 5H), 1.43-1.38 (m, 1H).

### Compound 63:

### (2-nitro-phenyl)-(2-pyridin-2-yl-ethyl)-amine

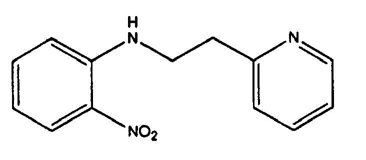

Compound 63 was obtained as an orange oil from 2-(2-aminoethyl)-pyridine and 2-fluoronitrobenzene by method H.

### Compound 64:

### 1-(2-Pyridin-2-yl-ethyl)-1,4-dihydro-quinoxaline-2,3-dione

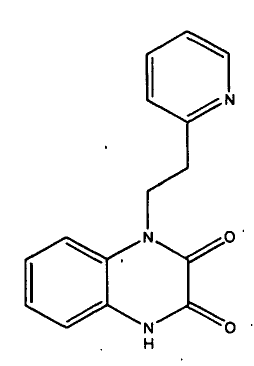

Compound 64 was obtained as a gray solid from compound 63 by method I.

### Compound 65:

### 3-Chloro-1-(2-pyridin-2-yl-ethyl)-1H-quinoxalin-2-one

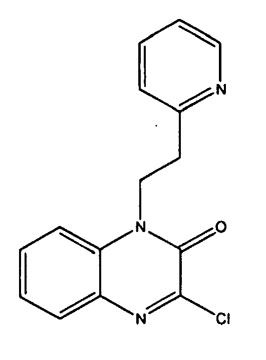

Compound 65 was obtained as a dark solid from compound 64 by method B.

### Compound 66:

### 4-[4-(2-pyridin-2-yl-ethyl)-3-oxo-3,4-dihydro-quinoxalin-2ylamino)]-N-thiophen-2-ylmethyl-benzamide

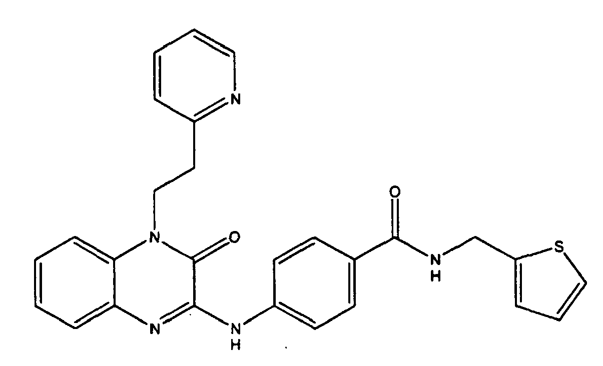

Compound 66 was obtained as a white solid from compound 65 and 4-amino-N-thiophen-2ylmethyl-benzamide by method C. ¹H NMR (DMSO) δ 9.68 (s, 1H), 9.02 (t, J=5.8 Hz, 1H), 8.53 (m, 1H), 8.26 (d, J=8.9 Hz, 2H), 7.88 (d, J=8.7 Hz, 2H), 7.72 (m, 1H), 7.62 (m, 1H), 7.54 (m, 1H), 7.40-7.23 (m, 5H), 7.03 (m, 1H), 6.67 (m, 1H), 4.66 (m, 4H), 3.17 (t, J=7.3 Hz, 2H).

### Compound 67:

### N,N-Di methyl-N-(2-nitro-phenyl)-ethane-1,2-diamine

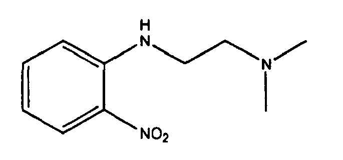

Compound 67 was obtained as an orange oil from 2-fluoronitrobenzene and N,N-dimethyl-ethylenediamine by method H.

### Compound 68:

### 1-(2-dimethylamino-ethyl)-1,4-dihydro-quinoxaline-2,3-dione

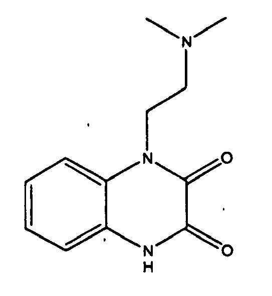

Compound 68 was obtained as a gray solid from compound 67 by method I.

### Compound 69:

### 3-Chloro-1-(2-dimethylamino-ethyl)-1H-quinoxalin-2-one

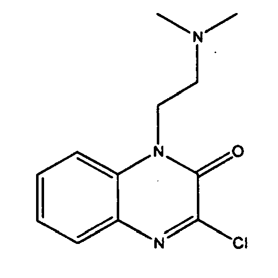

Compound 69 was obtained as a hydrochloride salt from compound 68 by method B.

### Compound 70:

### 4-[4-(2-dimethylamino-ethyl)-3-oxo-3,4-dihydro-quinoxalin-2ylamino)]-N-thiophen-2-ylmethyl-benzamide

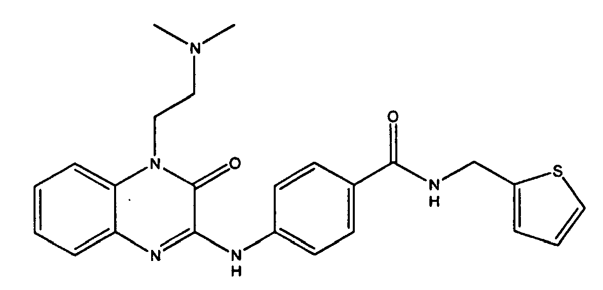

Compound 70 was obtained from compound 69 and 4-amino-N-thiophen-2ylmethyl-benzamide by method C as a trifluoroacetic acid salt after purification by reverse phase HPLC. ¹H NMR (DMSO) δ 9.76 (s, 1H), 9.20 (bs, 1H), 9.02 (t, J=5.8, 1H), 8.28 (d, J=8.8 Hz, 2H), 7.90 (d, J=8.8 Hz, 2H), 7.65 (dd, J=7.48, 1.9, 1H), 7.56 (d, J=7.89 Hz, 1H), 7.43-7.33 (m, 3H), 7.03 (d, J=2.5 Hz, 1H), 7.03 (d, J=2.5 Hz, 1H), 6.96 (dd, J= 5, 3.5 Hz, 1H), 4.70 (appt, J=5.6 Hz, 2H), 4.64 (d, J=5.6 Hz, 2H), 3.50 (d, J=4.6 Hz, 2H), 2.97 (s, 3H), 2.95 (s, 3H).

### Compound 71:

### (2-Nitro-phenylamino)-acetic acid tert-butyl ester

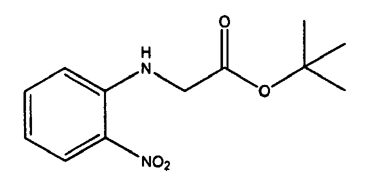

Compound 71 was obtained as an orange oil from 2-fluoronitrobenzene and glycine *tert-*butyl ester by method H.

### Compound 72:

### (2-Amino-phenylamino)-acetic acid tert-butyl ester

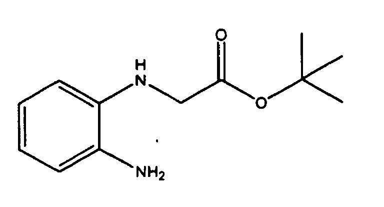

Compound 72 was obtained as a dark solid from compound 71 by method F.

### Compound 73:

### (2,3-dioxo-3,4-dihydro-2H-quinoxalin-1-yl)-acetic acid tert-butyl ester

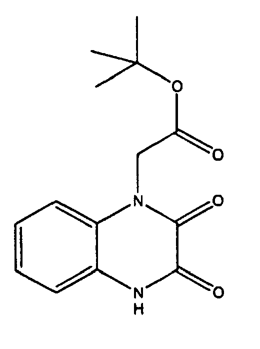

Compound 73 was obtained as a white solid from compound 72 by method A.

### Compound 74:

### (3-Chloro-2-oxo-2H-quinoxalin-1-yl)-acetic acid tert-butyl ester

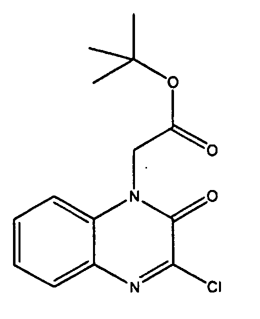

Compound 74 was obtained as a dark solid from compound 73 by method B.

### Compound 75:

### (2-Oxo-3-{4-[(thiophen-2ylmethyl)-carbamoyl]-phenylamino}-2H-quinoxalin-1-yl)-acetic acid

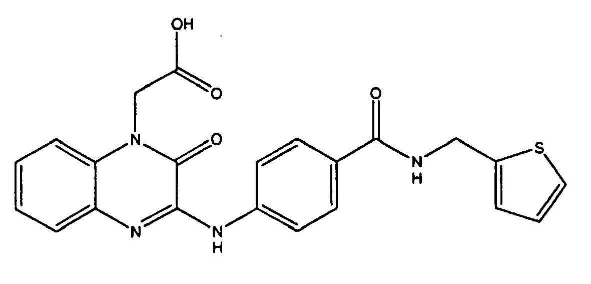

Compound 75 was obtained as a white solid from compound 74 and 4-amino-N-thiophen-2ylmethyl-benzamide by method C, followed by treatment with trifluoroacetic acid overnight at room temperature. ¹H NMR (DMSO) δ 9.77 (s, 1H), 9.04 (t, J=6.1 Hz, 1H), 8.25 (d, J=8.78 Hz, 2H), 7.90 (d, J=8.78 Hz, 2H), 7.41 (d, J=11.49 Hz, 1H), 7.38-7.30 (m, 4H), 7.04 (appt, J=2.4, 1 Hz, 1H), 6.97 (dd, J=5.1, 3.6 Hz, 1H), 5.09 (s, 2H), 4.65 (d, J=5.75 Hz, 2H).

### Compound 76:

### 2-Chloro-3-methoxy-quinoxaline

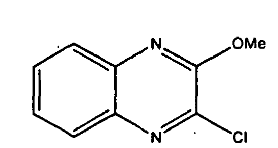

To a solution of 2,3-dichloro-quinoxaline (500 mg, 2.51 mmol) in THF (5mL) at 0°C was added sodium methoxide (0.57 mL of a 25% solution in methanol, 2.51 mmol) dropwise. The reaction was stirred at 0oc for 3.0 min then warmed to room temperature or 1h. The reaction mixture was diluted with dichloromethane, washed with brine, dried (MgSO₄), and concentrated *in vacuo* to give compound 76 as a yellow solid.

### Compound 77:

### 4-(3-oxo-3,4-dihydro-quinoxalin-2ylamino)-N-thiophen-2-ylmethyl-benzamide

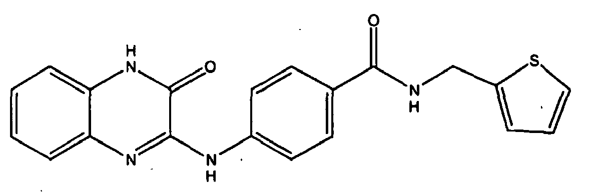

Compound 77 was obtained as a white solid from compound 76 by method D. ¹H NMR (DMSO) δ 12.49 (bs, 1H), 9.64 (s, 1H), 9.01 (t, J=5.8 Hz, 1H), 8.26 (d, J=8.8 Hz, 2H), 7.88 (d, J=8.8 Hz, 2H), 7.57-7.54 (m, 1H), 7.38 (m, 1H), 7.28-7.20 (m, 3H), 7.02 (m, 1H), 6.97 (m, 1H), 4.63 (d,J=5.8 Hz, 2H).

### Compound 78:

### N-Benzyl-4-(4-Methyl-3-oxo-3,4-dihydro-quinoxalin-2ylamino)-benzamide

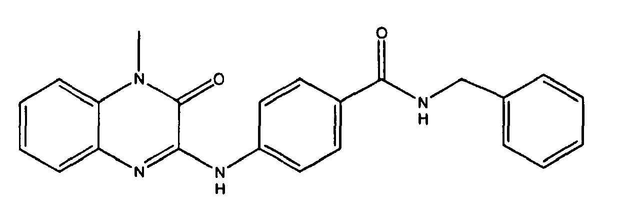

Compound 78 was obtained as a white solid from benzylamine and compound 3 by method D. ¹H NMR (DMSO) δ 9.70 (s, 1H), 8.93 (t, J=5.9 Hz, 1H), 8.26 (d, J=9.0 Hz, 2H), 7.92 (d, J=9.0 Hz, 2H), 7.61 (d, J=7.7 Hz, 1H), 7.52-7.21 (m, 8H), 4.49 (d, J=5.9 Hz, 2H), 3.71 (s, 3H).

### Biological Example 1

### Enzyme Inhibition Assay:

In 96-well clear plates, glycogen phosphorylase (from rabbit muscle, Sigma P1261) was incubated in a buffer (50 mM HEPES, pH 7.2, 100 mM KC1, 2.5 mM EGTA, and 2.5 mM MgCl₂) containing 1 mg/ml glycogen, 0.25 mM glucose 1-phosphate, and test compound for 15-30 minutes at room temperature (total volume is 100 mL). All samples contain 2% DMSO, and the background lacks enzyme but contains all substrates. The reaction was stopped by the addition of 150 mL of a 1N HCl solution containing 10 mg/ml ammonium molybdate and 0.38 mg/ml malachite green. After a 15-minute incubation period to allow the colored reaction (a quantitative measurement of inorganic phosphate) to develop, the colored wells were read at 650 nm. Data was reported as percent inhibition (+/- 10%) of enzymatic activity at a specified concentration.

| Compound | % inhibition | IC50 (µM, +/-.05) |
|---|---|---|
| 4 | 98% @ 20µM | 0.1 |
| 10 | 100% @ 20µM | 0.11 |
| 7 | 92% @ 20µM | 0.12 |
| 11 | 99% @ 20µM | 0.12 |
| 28 | 100% @ 20µM | 0.12 |
| 46 | 72% @ 20µM | 0.14 |
| 77 | 98% @ 20µM | 0.16 |
| 35 | 94% @ 20µM | 0.19 |
| 31 | 99% @ 20µM | 0.19 |
| 5 | 93% @ 20µM | 0.2 |
| 55 | 97% @ 20µM | 0.26 |
| 41 | 70% @ 20µM | 0.28 |
| 51 | 99% @ 20µM | 0.3 |
| 36 | 96% @ 20µM | 0.32 |
| 56 | 99% @ 20µM | 0.35 |
| 62 | 95% @ 20µM | 0.42 |
| 29 | 100% @ 20µM | 0.46 |
| 6 | 86% @ 20µM | 0.48 |
| 66 | 74% @ 20µM | 0.58 |
| 78 | 96% @ 20µM | 0.69 |
| 14 | 65% @ 20µM | 0.7 |
| 70 | 99% @ 20µM | 0.7 |
| 9 | 93% @ 20µM | 0.71 |
| 15 | 92% @ 20µM | 0.73 |
| 16 | 97% @ 20µM | 0.8 |
| 30 | 90% @ 20µM | 0.83 |
| 8 | 95% @ 20µM | 0.89 |
| 12 | 68% @ 20µM | 1.3 |
| 17 | 70% @ 20µM | 1.5 |
| 57 | 89% @ 20µM | 1.8 |
| 23 | 82% @ 20µM | 2.5 |
| 22 | 92% @ 20µM | 2.5 |
| 18 | 80% @ 20µM | 2.5 |
| 21 | 40% @ 20µM | 5 |
| 19 | 77% @ 20µM | 5 |
| 75 | 77% @ 20µM | 5.7 |

### G. Other Embodiments

The advantages and features of the invention will be apparent to one of ordinary skill from the disclosure herein and the appended claims. Other variations, modifications, and adaptations known or within the ability of one of ordinary skill in the art are contemplated as within the scope of the invention as disclosed herein.

## Claims

1. A compound of formula (I): 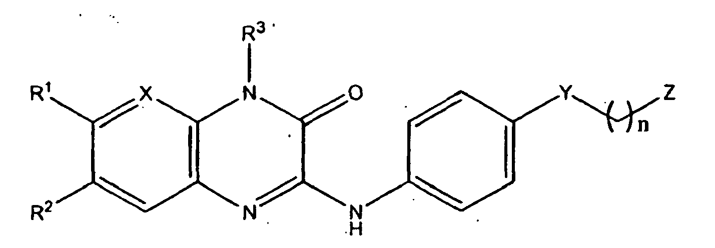 wherein R¹ is H, C₁₋₆ alkyl, or halo;
R² is H or halo;
R³ is H, C₁₋₆ alkyl, X is N or CH;
Y is a covalent bond, -NHCO- or -CONH-;
Z is phenyl or a 5 or 6-membered heterocyclyl with between 1 and 2 heteroatoms independently selected from N, O, and S; and
n is 0, 1 or 2;
or a pharmaceutically acceptable salt, ester, amide, hydrate, or solvate thereof.

2. A compound of claim 1 , wherein R¹ is H.

3. A compound of claim 1, wherein R² is H.

4. A compound of claim 1, wherein R³ is H or C₁₋₃ alkyl.

5. A compound of claim 1, wherein n is 0 or 1.

6. A compound of claim 1, wherein Y is -CONH-.

7. A compound of claim 1, wherein R³ is an alkyl substituted with hydroxy, carboxy, dimethylamino, piperidinyl, pyridinyl, or morpholinyl.

8. A compound of claim 1, wherein Z is selected from phenyl, furanyl, thiophenyl, isoxazolyl, pyridazyl, pyrazolyl, N-methyl-pyrazolyl, pyrimidyl, pyrrolyl, N-methyl-pyrrole, imidazolyl, pyridyl, oxazolyl, and thiazolyl.

9. A compound of claim 8, wherein Z is selected from furanyl, thiophenyl, isoxazolyl, and pyrazolyl.

10. A compound of claim 1, wherein R¹ is H; R² is H; and R³ is H or C₁₋₂ alkyl.

11. A compound of claim 1, wherein n is 0 or 1; and Y is -CONH-.

12. A compound of claim 10, wherein Z is selected from furanyl, thiophenyl, isoxazolyl, pyridazyl, pyrazolyl, pyrimidyl, pyrrolyl, imidazolyl, pyridyl, oxazolyl, and thiazolyl.

13. A compound of claim 12, wherein n is 0 or 1.

14. A compound of claim 1 selected from:
4-(4-Ethyl-3-oxo-3,4-dihydro-quinoxalin-2ylamino)-N-thiophen-2-ylmethyl-benzamide;
4-(4-Methyl-3-oxo-3,4-dihydro-quinoxalin-2ylamino)-N-(1*H*-pyrazol-3-yl)-benzamide;
N-Isoxazol-3-yl-4-[4-(2-Morpholin-4-yl-ethyl)-3-oxo-3,4-dihydro-quinoxalin-2ylamino]-benzamide;
4-(4,6-Dimethyl-3-oxo-3,4-dihydro-quinoxalin-2ylamino)-N-thiophen-2-ylmethyl-benzamide;
4-[4-(2-hydroxy-ethyl)-3-oxo-3,4-dihydro-quinoxalin-2ylamino)]-N-thiophen-2-ylmethyl-benzamide;
N-Isoxazol-3-yl-4-(4-Methyl-3-oxo-3,4-dihydro-pyrido[2,3-b]pyrazin-2-ylamino)-benzamide;
4-[4-(2-morpholin-4-yl-ethyl)-3-oxo-3,4-dihydro-quinoxalin-2ylamino)]-N-thiophen-2-ylmethyl-benzamide; and
4-[4-(2-piperidin-1-yl-ethyl)-3-oxo-3,4-dihydro-quinoxalin-2ylamino)]-N-thiophen-2-ylmethyl-benzamide.

15. A compound of claim 1 selected from:
N-Isoxazol-3-yl-4-(4-Methyl-3-oxo-3,4-dihydro-quinoxalin-2ylamino)-benzamide;
4-(4-Methyl-3-oxo-3,4-dihydro-quinoxalin-2ylamino)-N-thiophen-2-ylmethyl-benzamide;
N-Isoxazol-5-yl-4-(4-Methyl-3-oxo-3,4-dihydro-quinoxalin-2ylamino)-benzamide;
4-(4-Methyl-3-oxo-3,4-dihydro-quinoxalin-2ylamino)-N-furan-2-ylmethyl-benzamide;
N-Isoxazol-3-yl-4-(4-Ethyl-3-oxo-3,4-dihydro-quinoxalin-2ylamino)-benzamide;
4-(7-Fluoro-4-methyl-3-oxo-3,4-dihydro-quinoxalin-2ylamino)-N-thiophen-2-ylmethyl-benzamide; and
4-(4-Methyl-3-oxo-3,4-dihydro-pyrido[2,3-b]pyrazin-2-ylamino)-N-thiophen-2-ylmethyl-benzamide..

16. A compound of claim 1 selected from:
Isoxazole-5-carboxylic acid [4-(4-Methyl-3-oxo-3,4-dihydro-quinoxalin-2ylamino)-phenyl]-amide;
I*s*oxazole-3-carboxylic acid [4-(4-Methyl-3-oxo-3,4-dihydro-quinoxalin-2ylamino) phenyl]-amide; and
*N*-[4-(4-Methyl-3-oxo-3,4-dihydro-quinoxalin-2ylamino)-phenyl]-nicotinamide.

17. A compound of claim 1 selected from:
1-Methyl-3-[4-(1H-pyrro]-2-yl)-phenylamino]-1*H*-quinoxalin-2-one; and
3-(4-imidazol-1-yl-phenylamino)-1-methyl-1*H*-quinoxalin-2-one.

18. A compound of claim 1 selected from:
N-Isoxazol-3-yl-4-(7-Fluoro-4-methyl-3-oxo-3,4-dihydro-quinoxalin-2ylamino)-benzamide;
4-(6-Fluoro-4-methyl-3-oxo-3,4-dihydro-quinoxalin,2ylamino)-N-thiophen-2-ylmethyl-benzamide;
N-Isoxazol-3-yl-4-(6-Fluoro-4-methyl-3-oxo-3,4-dihydro-quinoxalin-2ylamino)-benzamide;
N-Isoxazol-3-yl-4-(6-Chloro-4-methyl-3-oxo-3,4-dihydro-quinoxalin-2ylamino)-benzamide;
N-Isoxazol-3-yl-4-(7-Chloro-4-methyl-3-oxo-3,4-dihydro-quinoxalin-2ylamino)-benzamide;
4-(6-Chloro-4-methyl-3-oxo-3,4-dihydro-quinoxalin-2ylamino)-N-thiophen-2-ylmethyl-benzamide;
4-(7-Chloro-4-methyl-3-oxo-3,4-dihydro-quinoxalin-2ylamino)-N-thiophen-2-ylmethyl-benzamide;
N-lsoxazol-3-yl-4-[4-(2-piperidin- I -yl-ethyl)-3-oxo-3,4-dihydro-quinoxalin-2ylamino]-benzamide;
4-(4-Ethyl-3-oxo-3,4-dihydro-pyrido[2,3-b]pyrazin-2-ylamino)-N-thiophen-2-ylmethyl-benzamide;
N-lsoxazol-3-yl-4-(4-ethyl-3-oxo-3,4-dihydro-pyrido[2,3-b]pyrazin-2-ylamino)-benzamide;
N-lsoxazol-3-yl-4-[7-fluoro-4-(2-Morpholin-4-yl-ethyl)-3-oxo-3,4-dihydro-quinoxalin-2ylamino]-benzamide;
4-[7-Fluoro-4-(2-piperidin-1-yl-ethyl)-3-oxo-3,4-dihydro-quinoxalin-2ylamino)]-N-thiophen-2-ylmethyl-benzamide;
4-(4-Methyl-3-oxo-3,4-dihydro-quinoxalin-2ylamino)-N-oxazol-2-yl-benzamide;
N-Furan-2-yl-4-(4-Methyl-3-oxo-3,4-dihydro-quinoxalin-2ylamino)-benzamide;
4-(4-Methyl-3-oxo-3,4-dihydro-quinoxalin-2ylamino)-N-thiophen-2-yl-benzamide;
4-(4-Methyl-3-oxo-3,4-dihydro-quinoxalin-2ylaminopN-(1*H*-pyrrol-3-yl)-benzamide;
4-(4-Methyl-3-oxo-3,4-dihydro-quinoxalin-2ylamino)-N-(1-methyl-1*H*-pyrrol-3-yl)-benzamide;
4-(4-Methyl-3-oxo-3,4-dihydro-quinoxalin-2ylamino)-N-(pyrimidin-2-yl)-benzamide;
N-(1*H*-Imidazol-2-yl)-4-(4-methyl-3-oxo-3,4-dihydro-quinoxalin-2ylamino)-benzamide;
4-[4-(2-pyrrolidin-1-yl-ethyl)-3-oxo-3,4-dihydro-quinoxalin-2ylamino)]-N-thiophen-2-ylmethyl-benzamide; and
N-lsoxazol-3-ylmethyl-4-(4-methyl-3-oxo-3,4-dihydro-quinoxalin-2ylamino)-benzamide.

19. A pharmaceutical composition, comprising a compound of claim 1, 14, 15, 16, 17, or 18 and a pharmaceutically acceptable carrier.

20. Use of a compound as defined in claim 1, in the manufacture of a composition for the treatment of diabetes.

21. Use according to claim 20, wherein said diabetes is Type 1 or Type II diabetes.

22. Use of a compound as defined in claim 1, in the manufacture of a composition for the treatment of obesity.

## Patentansprüche

1. Verbindung von Formel (I): 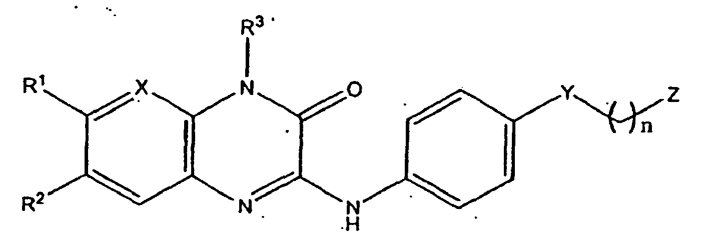 worin R¹ H, C₁₋₆-Alkyl oder Halo ist;
R² H oder Halo ist;
R³ H, C₁₋₆-Alkyl ist, X N oder CH ist;
Y eine kovalente Bindung, -NHCO- oder -CONH- ist;
Z Phenyl oder ein 5- oder 6-gliedriges Heterocyclyl mit zwischen 1 und 2 Heteroatomen, unabhängig ausgewählt aus N, O und S, ist; und
n 0, 1 oder 2 ist;
oder ein pharmazeutisch annehmbares Salz, Ester, Amid, Hydrat oder Solvat davon.

2. Verbindung nach Anspruch 1, **dadurch gekennzeichnet, daß** R¹ H ist.

3. Verbindung nach Anspruch 1, **dadurch gekennzeichnet, daß** R² H ist.

4. Verbindung nach Anspruch 1, **dadurch gekennzeichnet, daß** R³ H oder C₁₋₃-Alkyl ist.

5. Verbindung nach Anspruch 1, **dadurch gekennzeichnet, daß** n 0 oder 1 ist.

6. Verbindung nach Anspruch 1, **dadurch gekennzeichnet, daß** Y -CONH- ist.

7. Verbindung nach Anspruch 1, **dadurch gekennzeichnet, daß** R³ ein Alkyl ist, substituiert mit Hydroxy, Carboxy, Dimethylamino, Piperidinyl, Pyridinyl oder Morpholinyl.

8. Verbindung nach Anspruch 1, **dadurch gekennzeichnet, daß** Z ausgewählt ist aus Phenyl, Furanyl, Thiophenyl, Isoxazolyl, Pyridazyl, Pyrazolyl, N-Methylpyrazolyl, Pyrimidyl, Pyrrolyl, N-Methylpyrrol, Imidazolyl, Pyridyl, Oxazolyl und Thiazolyl.

9. Verbindung nach Anspruch 8, **dadurch gekennzeichnet, daß** Z ausgewählt ist aus Furanyl, Thiophenyl, Isoxazolyl und Pyrazolyl.

10. Verbindung nach Anspruch 1, **dadurch gekennzeichnet, daß** R¹ H ist; R² H ist; und R³ H oder C₁₋₂-Alkyl ist.

11. Verbindung nach Anspruch 1, **dadurch gekennzeichnet, daß** n 0 oder 1 ist; und Y -CONH- ist.

12. Verbindung nach Anspruch 10, **dadurch gekennzeichnet, daß** Z ausgewählt ist aus Furanyl, Thiophenyl, Isoxazolyl, Pyridazyl, Pyrazolyl, Pyrimidyl, Pyrrolyl, Imidazolyl, Pyridyl, Oxazolyl und Thiazolyl.

13. Verbindung nach Anspruch 12, **dadurch gekennzeichnet, daß** n 0 oder 1 ist.

14. Verbindung nach Anspruch 1, ausgewählt aus:
4-(4-Ethyl-3-oxo-3,4-dihydrochinoxalin-2-ylamino)-N-thiophen-2-ylmethylbenzamid;
4-(4-Methyl-3-oxo-3,4-dihydrochinoxalin-2-ylamino)-N-(1H-pyrazol-3-yl)-benzamid;
N-Isoxazol-3-yl-4-[4-(2-morpholin-4-ylethyl)-3-oxo-3,4-dihydrochinoxalin-2-ylamino]-benzamid;
4-(4,6-Dimethyl-3-oxo-3,4-dihydrochinoxalin-2-ylamino)-N-thiophen-2-ylmethylbenzamid;
4-[4-(2-Hydroxyethyl)-3-oxo-3,4-dihydrochinoxalin-2-ylamino)]-N-thiophen-2-ylmethylbenzamid;
N-Isoxazol-3-yl-4-(4-methyl-3-oxo-3,4-dihydropyrido[2,3-b]pyrazin-2-ylamino)-benzamid;
4-[4-(2-Morpholin-4-ylethyl)-3-oxo-3,4-dihydrochinoxalin-2-ylamino)]-N-thiophen-2-ylmethylbenzamid; und
4-[4-(2-Piperidin-1-ylethyl)-3-oxo-3,4-dihydrochinoxalin-2-ylamino)]-N-thiophen-2-ylmethylbenzamid.

15. Verbindung nach Anspruch 1, ausgewählt aus:
N-Isoxazol-3-yl-4-(4-methyl-3-oxo-3,4-dihydrochinoxalin-2-ylamino)-benzamid;
4-(4-Methyl-3-oxo-3,4-dihydrochinoxalin-2-ylamino)-N-thiophen-2-ylmethylbenzamid;
N-Isoxazol-5-yl-4-(4-methyl-3-oxo-3,4-dihydrochinoxalin-2-ylamino)-benzamid;
4-(4-Methyl-3-oxo-3,4-dihydrochinoxalin-2-ylamino)-N-furan-2-ylmethylbenzamid;
N-Isoxazol-3-yl-4-(4-ethyl-3-oxo-3,4-dihydrochinoxalin-2-ylamino)-benzamid;
4-(7-Fluor-4-methyl-3-oxo-3,4-dihydrochinoxalin-2-ylamino)-N-thiophen-2-ylmethylbenzamid; und
4-(4-Methyl-3-oxo-3,4-dihydropyridio[2,3-b]pyrazin-2-ylamino)-N-thiophen-2-ylmethylbenzamid.

16. Verbindung nach Anspruch 1, ausgewählt aus:
Isoxazol-5-carbonsäure-[4-(4-methyl-3-oxo-3,4-dihydrochinoxalin-2-ylamino)-phenyl]-amid;
Isoxazol-3-carbonsäure-[4-(4-methyl-3-oxo-3,4-dihydrochinoxalin-2-ylamino)-phenyl]-amid; und
N-[4-(4-Methyl-3-oxo-3,4-dihydrochinoxalin-2-ylamino)-phenyl]-nicotinamid.

17. Verbindung nach Anspruch 1, ausgewählt aus:
1-Methyl-3-[4-(1H-pyrrol-2-yl)-phenylamino]-1H-chinoxalin-2-on; und
3-(4-Imidazol-1-ylphenylamino)-1-methyl-1H-chinoxalin-2-on.

18. Verbindung nach Anspruch 1, ausgewählt aus:
N-Isoxazol-3-yl-4-(7-fluor-4-methyl-3-oxo-3,4-dihydrochinoxalin-2-ylamino)-benzamid;
4-(6-Fluor-4-methyl-3-oxo-3,4-dihydrochinoxalin-2-ylamino)-N-thiophen-2-ylmethylbenzamid;
N-Isoxazol-3-yl-4-(6-fluor-4-methyl-3-oxo-3,4-dihydrochinoxalin-2-ylamino)-benzamid;
N-Isoxazol-3-yl-4-(6-chlor-4-methyl-3-oxo-3,4-dihydrochinoxalin-2-ylamino)-benzamid;
N-Isoxazol-3-yl-4-(7-chlor-4-methyl-3-oxo-3,4-dihydrochinoxalin-2-ylamino)-benzamid;
4-(6-Chlor-4-methyl-3-oxo-3,4-dihydrochinoxalin-2-ylamino)-N-thiophen-2-ylmethylbenzamid;
4-(7-Chlor-4-methyl-3-oxo-3,4-dihydrochinoxalin-2-ylamino)-N-thiophen-2-ylmethylbenzamid;
N-Isoxazol-3-yl-4-[4-(2-piperidin-1-ylethyl)-3-oxo-3,4-dihydrochinoxalin-2-ylamino]-benzamid;
4-(4-Ethyl-3-oxo-3,4-dihydropyrido[2,3-b]pyrazin-2-ylamino)-N-thiophen-2-ylmethylbenzamid;
N-Isoxazol-3-yl-4-(4-ethyl-3-oxo-3,4-dihydropyrido[2,3-b]pyrazin-2-ylamino)-benzamid;
N-Isoxazol-3-yl-4-(7-fluor-4-(2-morpholin-4-ylethyl)-3-oxo-3,4-dihydrochinoxalin-2-ylamino]-benzamid;
4-[7-Fluor-4-(2-piperidin-1-ylethyl)-3-oxo-3,4-dihydrochinoxalin-2-ylamino)]-N-thiophen-2-ylmethylbenzamid;
4-(4-Methyl-3-oxo-3,4-dihydrochinoxalin-2-ylamino)-N-oxazol-2-ylbenzamid;
N-Furan-2-yl-4-(4-methyl-3-oxo-3,4-dihydrochinoxalin-2-ylamino)-benzamid;
4-(4-Methyl-3-oxo-3,4-dihydrochinoxalin-2-ylamino)-N-thiophen-2-ylbenzamid;
4-(4-Methyl-3-oxo-3,4-dihydrochinoxalin-2-ylamino)-N-(1H-pyrrol-3-yl)-benzamid;
4-(4-Methyl-3-oxo-3,4-dihydrochinoxalin-2-ylamino)-N-(1 -methyl- H-pyrrol-3-yl)-benzamid ;
4-(4-Methyl-3-oxo-3,4-dihydrochinoxalin-2-ylamino)-N-(pyrimidin-2-yl)-benzamid;
N-(1H-Imidazol-2-yl)-4-(4-methyl-3-oxo-3,4-dihydrochinoxalin-2-ylamino)-benzamid;
4-[4-(2-Pyrrolidin-1-ylethyl)-3-oxo-3,4-dihydrochinoxalin-2-ylamino)]-N-thiophen-2-ylmethylbenzamid ; und
N-Isoxazol-3-ylmethyl-4-(4-methyl-3-oxo-3,4-dihydrochinoxalin-2-ylamino)-benzamid.

19. Pharmazeutische Zusammensetzung, die eine Verbindung nach Anspruch 1, 14, 15, 16, 17 oder 18 und einen pharmazeutisch annehmbaren Trägerstoff umfaßt.

20. Verwendung einer Verbindung, wie definiert in Anspruch 1, bei der Herstellung einer Zusammensetzung zur Behandlung von Diabetes.

21. Verwendung nach Anspruch 20, **dadurch gekennzeichnet, daß** besagte Diabetes Diabetes Typ I oder Typ II ist.

22. Verwendung einer Verbindung, wie definiert in Anspruch 1, bei der Herstellung einer Zusammensetzung zur Behandlung von Fettleibigkeit.

## Revendications

1. Composé de formule (I) : 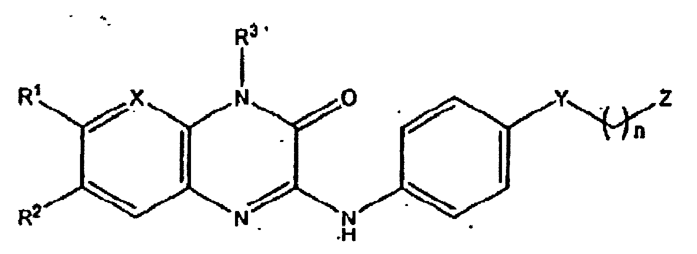 dans laquelle R¹ représente H, un groupe alkyle en C₁ à C₆ ou halogéno ;
R² représente H ou un groupe halogéno ;
R³ représente H, un groupe alkyle en C₁ à C₆, X représente N ou CH ;
Y représente une liaison covalente, -NHCO- ou -CONH- ;
Z représente un groupe phényle ou un groupe hétérocyclyle de 5 ou 6 membres avec entre 1 et 2 hétéroatomes choisis indépendamment parmi N, O et S ; et
n vaut 0, 1 ou 2 ;
ou un sel, un ester, un amide, un hydrate ou un solvate pharmaceutiquement acceptable de celui-ci.

2. Composé selon la revendication 1, où R¹ représente H.

3. Composé selon la revendication 1, où R² représente H.

4. Composé selon la revendication 1, où R³ représente H ou un groupe alkyle en C₁ à C₃.

5. Composé selon la revendication 1, où n vaut 0 ou 1.

6. Composé selon la revendication 1, où Y représente -CONH-.

7. Composé selon la revendication 1, où R³ représente un groupe alkyle substitué par un groupe hydroxy, carboxy, diméthylamino, pipéridinyle, pyridinyle ou morpholinyle.

8. Composé selon la revendication 1, où Z est choisi parmi les groupes phényle, furanyle, thiophényle, isoxazolyle, pyridazyle, pyrazolyle, N-méthyl-pyrazolyle, pyrimidyle, pyrrolyle, N-méthyl-pyrrole, imidazolyle, pyridyle, oxazolyle et thiazolyle.

9. Composé selon la revendication 8, où Z est choisi parmi les groupes furanyle, thiophényle, isoxazolyle et pyrazolyle.

10. Composé selon la revendication 1, où R¹ représente H ; R² représente H ; et R³ représente H ou un groupe alkyle en C₁ à C₂.

11. Composé selon la revendication 1, où n vaut 0 ou 1 ; et Y représente -CONH-.

12. Composé selon la revendication 10, où Z est choisi parmi les groupes furanyle, thiophényle, isoxazolyle, pyridazyle, pyrazolyle, pyrimidyle, pyrrolyle, imidazolyle, pyridyle, oxazolyle et thiazolyle.

13. Composé selon la revendication 12, où n vaut 0 ou 1.

14. Composé selon la revendication 1, choisi parmi :
le 4-(4-éthyl-3-oxo-3,4-dihydro-quinoxalin-2-yl-amino)-N-thiophén-2-ylméthyl-benzamide ;
le 4-(4-méthyl-3-oxo-3,4-dihydro-quinoxalin-2-yl-amino)-N-(1H-pyrazol-3-yl)-benzamide ;
le N-isoxazol-3-yl-4-[4-(2-morpholin-4-yl-éthyl)-3-oxo-3,4-dihydro-quinoxalin-2-ylamino]-benzamide ;
le 4-(4,6-diméthyl-3-oxo-3,4-dihydro-quinoxalin-2-ylamino)-N-thiophén-2-ylméthyl-benzamide ;
le 4-[4-(2-hydroxy-éthyl)-3-oxo-3,4-dihydro-quinoxalin-2-ylamino)]-N-thiophén-2-ylméthylbenzamide ;
le N-isoxazol-3-yl-4-(4-méthyl-3-oxo-3,4-dihydro-pyrido[2,3-b]pyrazin-2-ylamino)-benzamide ;
le 4-[4-(2-morpholin-4-yl-éthyl)-3-oxo-3,4-dihydro-quinoxalin-2-ylamino)]-N-thiophén-2-ylméthylbenzamide ; et
le 4-[4-(2-pipéridin-1-yl-éthyl)-3-oxo-3,4-dihydro-quinoxalin-2-ylamino)]-N-thiophén-2-ylméthylbenzamide.

15. Composé selon la revendication 1, choisi parmi :
le N-isoxazol-3-yl-4-(4-méthyl-3-oxo-3,4-dihydro-quinoxalin-2-ylamino)-benzamide ;
le 4-(4-méthyl-3-oxo-3,4-dihydro-quinoxalin-2-yl-amino)-N-thiophén-2-ylméthyl-benzamide ;
le N-isoxazol-5-yl-4-(4-méthyl-3-oxo-3,4-dihydro-quinoxalin-2-ylamino)-benzamide ;
le 4-(4-méthyl-3-oxo-3,4-dihydro-quinoxalin-2-yl-amino)-N-furan-2-ylméthyl-benzamide ;
le N-isoxazol-3-yl-4-(4-éthyl-3-oxo-3,4-dihydro-quinoxalin-2-ylamino)-benzamide ;
le 4-(7-fluoro-4-méthyl-3-oxo-3,4-dihydroquinoxalin-2-ylamino)-N-thiophén-2-ylméthylbenzamide ; et
le 4-(4-méthyl-3-oxo-3,4-dihydro-pyrido[2,3-b]-pyrazin-2-ylamino)-N-thiophén-2-ylméthyl-benzamide.

16. Composé selon la revendication 1, choisi parmi:
le [4-(4-méthyl-3-oxo-3,4-dihydro-quinoxalin-2-ylamino)-phényl]-amide de l'acide isoxazole-5-carboxylique ;
le [4-(4-méthyl-3-oxo-3,4-dihydro-quinoxalin-2-ylamino)-phényl]-amide de l'acide isoxazole-3-carboxylique ; et
le *N*-[4-(4-méthyl-3-oxo-3,4-dihydro-quinoxalin-2-ylamino)-phényl]-nicotinamide.

17. Composé selon la revendication 1, choisi parmi:
la 1-méthyl-3-[4-(1H-pyrrol-2-yl)-phénylamino]-1*H*-quinoxalin-2-one ; et
la 3-(4-imidazol-1-yl-phénylamino)-1-méthyl-1*H-*quinoxalin-2-one.

18. Composé selon la revendication 1, choisi parmi :
le N-isoxazol-3-yl-4-(7-fluoro-4-méthyl-3-oxo-3, 4-dihydro-quinoxalin-2-ylamino)-benzamide ;
le 4-(6-fluoro-4-méthyl-3-oxo-3,4-dihydro-quinoxalin-2-ylamino)-N-thiophén-2-ylméthyl-benzamide ;
le N-isoxazol-3-yl-4-(6-fluoro-4-méthyl-3-oxo-3, 4-dihydro-quinoxalin-2-ylamino)-benzamide ;
le N-isoxazol-3-yl-4-(6-chloro-4-méthyl-3-oxo-3, 4-dihydro-quinoxalin-2-ylamino)-benzamide ;
le N-isoxazol-3-yl-4-(7-chloro-4-méthyl-3-oxo-3, 4-dihydro-quinoxalin-2-ylamino)-benzamide ;
le 4-(6-chloro-4-méthyl-3-oxo-3,4-dihydro-quinoxalin-2-ylamino)-N-thiophén-2-ylméthyl-benzamide ;
le 4-(7-chloro-4-méthyl-3-oxo-3,4-dihydro-quinoxalin-2-ylamino)-N-thiophén-2-ylméthyl-benzamide ;
le N-isoxazol-3-yl-4-[4-(2-pipéridin-1-yl-éthyl)-3-oxo-3,4-dihydro-quinoxalin-2-ylamino]-benzamide ;
le 4-(4-éthyl-3-oxo-3,4-dihydro-pyrido[2,3-b]-pyrazin-2-ylamino)-N-thiophén-2-ylméthyl-benzamide ;
le N-isoxazol-3-yl-4-(4-éthyl-3-oxo-3,4-dihydro-pyrido[2,3-b]pyrazin-2-ylamino)-benzamide ;
le N-isoxazol-3-yl-4-[7-fluoro-4-(2-morpholin-4-yl-éthyl)-3-oxo-3,4-dihydro-quinoxalin-2-ylamino]-benzamide ;
le 4-[7-fluoro-4-(2-pipéridin-1-yl-éthyl)-3-oxo-3,4-dihydro-quinoxalin-2-ylamino]-N-thiophén-2-ylméthyl-benzamide ;
le 4-(4-méthyl-3-oxo-3,4-dihydro-quinoxalin-2-ylamino)-N-oxazol-2-yl-benzamide ;
le N-furan-2-yl-4-(4-méthyl-3-oxo-3,4-dihydroquino-xalin-2-ylamino)-benzamide ;
le 4-(4-méthyl-3-oxo-3,4-dihydro-quinoxalin-2-yl-amino)-N-thiophén-2-yl-benzamide ;
le 4-(4-méthyl-3-oxo-3,4-dihydro-quinoxalin-2-yl-amino)-N-(1*H*-pyrrol-3-yl)-benzamide ;
le 4-(4-méthyl-3-oxo-3,4-dihydro-quinoxalin-2-yl-amino)-N-(1-méthyl-1*H*-pyrrol-3-yl)-benzamide ;
le 4-(4-méthyl-3-oxo-3,4-dihydro-quinoxalin-2-yl-amino)-N-(pyrimidin-2-yl)-benzamide ;
le N-(1*H*-imidazol-2-yl)-4-(4-méthyl-3-oxo-3,4-dihydro-quinoxalin-2-ylamino)-benzamide ;
le 4-[4-(2-pyrrolidin-1-yl-éthyl)-3-oxo-3,4-dihydro-quinoxalin-2-ylamino)-N-thiophén-2-ylméthyl-benzamide ; et
le N-isoxazol-3-ylméthyl-4-(4-méthyl-3-oxo-3,4-dihydro-quinoxalin-2-ylamino]-benzamide.

19. Composition pharmaceutique, comprenant un composé selon la revendication 1, 14, 15, 16, 17 ou 18 et un support pharmaceutiquement acceptable.

20. Utilisation d'un composé selon la revendication 1, dans la fabrication d'une composition destinée au traitement du diabète.

21. Utilisation selon la revendication 20, où ledit diabète est un diabète de type I ou de type II.

22. Utilisation d'un composé selon la revendication 1, dans la fabrication d'une composition destinée au traitement de l'obésité.
